# EUROPEAN PATENT APPLICATION

(11) **EP 4 092 051 A1**
(43) Date of publication of application: **23.11.2022**
(21) Application number: 20940651.1
(22) Date of filing: 18.06.2020
(51) Int. Cl.: C07K 16/28, C12N 15/06, C12N 5/10, A61K 39/395

(54) **ANTI-SOLUBLE CD14 SUBTYPE ANTIBODY, KIT AND USE THEREOF**

(71) Applicant: Shenzhen Mindray Bio-Medical Electronics Co., Ltd, Shenzhen, Guangdong 518057 (CN)
(72) Inventor: YU, Lina, Shenzhen, Guangdong 518057 (CN); LI, Ke, Shenzhen, Guangdong 518057 (CN); HE, Jianwen, Shenzhen, Guangdong 518057 (CN)
(74) Representative: KIPA AB
(86) International application number: PCT/CN2020/096856
(87) International publication number: WO 2021/253335

(57) **Abstract**

An anti-soluble CD14 subtype monoclonal antibody or an antigen-binding antibody fragment thereof specifically recognizes an epitope composed of the amino acid sequence of SEQ ID No. 84. A kit for detecting the soluble CD14 subtype, a method for detecting the soluble CD14 subtype and corresponding use.

## Description

### TECHNICAL FIELD

The disclosure relates to an anti-soluble CD14 subtype monoclonal antibody or an antigen-binding antibody fragment thereof. The disclosure also relates to a kit for detecting a soluble CD14 subtype, a method for detecting a soluble CD14 subtype, and the corresponding use thereof.

### BACKGROUND

Sepsis is a life-threatening organ dysfunction caused by a dysregulated host response to infection. It is reported in literatures and materials that the incidence of sepsis is increasing year by year, and the fatality rate remains high, which has seriously threatened human life and health. According to the treatment guidelines for sepsis, early diagnosis is an effective way to improve the survival rate of patients with sepsis. Blood culture is the "gold standard" for diagnosis of sepsis; however, it has a low positive rate and a long culture time. Therefore, there is an urgent need to find biomarkers with high specificity and sensitivity for early diagnosis and accurate prognosis of sepsis.

The diagnostic criteria for sepsis are also constantly updated. The latest diagnostic criteria for sepsis (Sepsis 3.0) emphasize the infection and the resulting uncontrolled response and organ dysfunction in the patient's body. Although there are still many controversies about Sepsis 3.0, it reflects the progress of clinical research and diagnosis, as well as the complexity of sepsis and the importance of clinical diagnosis. Currently, in the diagnosis of sepsis, clinically widely used biomarkers are interleukin 6 (IL-6), C reactive protein (CRP) and procalcitonin (PCT). CRP is an acute phase protein synthesized by the liver. It has strong sensitivity to bacterial infection, but has low specificity, long half-life and no clear correlation with the progression of sepsis. The peak values of IL-6 appear early and can rise before clinical symptoms appear, which is beneficial for early diagnosis of sepsis, but has low specificity. PCT is widely used in the diagnosis of infection and sepsis. PCT is often not significantly elevated in certain types of infections, such as skin and soft tissue infections. PCT is not suitable for determining the prognosis of septic shock infection in perioperative abdominal infections. In addition, in some non-infectious diseases, PCT could show non-specific elevation. False-positive diagnoses are prone to occur in some cases such as trauma, surgery, and bums. Therefore, PCT is not a perfect marker for the diagnosis of infection and sepsis, and PCT alone is not reliable for the diagnosis of infection and sepsis.

CD14 is a high-affinity receptor for a lipopolysaccharide-lipopolysaccharide binding protein complex, and is anchored to the cell surface via glycosylphosphatidylinositol. CD14 exists mainly on the membrane surface of monocytes and macrophages. CD14 exists in the forms of membrane-bound CD14 (mCD14) and soluble CD14 (sCD14). Membrane-bound CD14 is mainly expressed on the surface of monocytes/macrophages. Soluble CD14 is distributed in the plasma and is the part of the LPS-LBF-CD14 complex released in blood after CD14 is shed from the cell membrane, which mainly mediates the response of endothelial cells and epithelial cells to LPS.

Soluble CD14 subtype (referred to as sCD14-ST or Presepsin for short), as a new biological indicator of sepsis, has potential application value in the diagnosis and prognosis of sepsis and in the guidance of the use of antimicrobial drugs in sepsis, and has become a hot spot in the research on markers of sepsis in recent years. Shirakawa et al. established an endotoxic shock model and a caecal ligation and perforation model (CLP model) in rabbits and found that the former did not result in an elevation in the concentration of Presepsin, whereas the CLP model resulted in a significant elevation. Therefore, it is believed that the elevation of Presepsin is resulted from phagocytosis caused by bacterial infection, rather than by physiological resistance to inflammation. Presepsin is an N-terminal fragment of sCD14 after being cleaved by proteases such as cathepsin D in the plasma, and has a relative molecular weight of about 13 kDa. At present, Presepsin is considered to have high sensitivity and specificity in diagnosis and prognosis of infections and sepsis, and could potentially guide the use of antimicrobial drugs in sepsis.

Therefore, attempts have been made so far on how to perform highly specific and sensitive diagnosis and accurate prognosis of sepsis.

### SUMMARY

Therefore, an objective of the disclosure is to provide an anti-soluble CD14 subtype monoclonal antibody or an antigen-binding antibody fragment thereof, which is suitable for detecting a soluble CD14 subtype in a sample. In addition, another objective of the disclosure is to provide a kit for detecting a soluble CD14 subtype, a method for detecting a soluble CD14 subtype, and the corresponding use thereof.

A first aspect of the disclosure relates to an anti-soluble CD14 subtype monoclonal antibody or an antigen-binding antibody fragment thereof, which specifically recognizes an epitope composed of the amino acid sequence of SEQ ID No. 84, where the anti-soluble CD14 subtype monoclonal antibody or the antigen-binding antibody fragment thereof comprises:
(i) heavy chain variable region (VH) complementarity determining regions (CDRs):
   VH CDR1: X₁X₂X₃MX₄;
   VH CDR2: RIX₅X₆DPX₇; and
   VH CDR3: X₈X₉X₁₀RX₁₁;
      and
(ii) light chain variable region (VL) complementarity determining regions (CDRs):
   VL CDR1: KX₁₂X₁₃X₁₄LA;
   VL CDR2: WX₁₅X₁₆; and
   VL CDR3: KQX₁₇X₁₈;
where X₁ to X₁₈ are one or more of the amino acid sequences listed below as options:
X₁ = any one of the amino acids;
X₂ = T or V; X₃ = A, Y or L; X₄ = Q or G;
X₅ = TYANG, DPMGG, SPASS, DPANG, or SPANS;
X₆ = GTKA, TTKS, NTKY, TTKS or TTKA;
X₇ = KFQA, TKFG, SQFA, MKFG or KFNS;
X₈ = F or G; X₉ = GG, GQ or CG; X₁₀ = YN, YV or KF; X₁₁ = FDY, FYY, FDC or TDC;
X₁₂ = SSAS, STAT, SSQS or STSS;
X₁₃ = LLKS, LLAS, LLSS or KASS;
X₁₄ = NRNIHL, RTRKHY, NRKHYL or YTNKAL;
X₁₅ = ATS, AST, QSS or ASK;
X₁₆ = RAS, LDS, LEA or RES;
X₁₇ = GYN, YNA, YLN or SYN;
X₁₈ = LWT, LNT, GNA or TWI.

A second aspect of the disclosure relates to a kit for detecting a soluble CD14 subtype, where the kit for detecting a soluble CD14 subtype comprises at least the anti-soluble CD14 subtype monoclonal antibody or the antigen-binding antibody fragment thereof according to the first aspect of the disclosure.

A third aspect of the disclosure relates to a method for detecting a soluble CD14 subtype, comprising:
mixing a sample to be tested with a solid phase support coated with a capture antibody, so that the capture antibody coated on the solid phase support binds to the soluble CD14 subtype in the sample to be tested;
washing the obtained mixture to remove unbound substances;
adding a labelled detection antibody to the washed mixture and mixing evenly, so that the detection antibody binds to the soluble CD14 subtype bound on the capture antibody to form a sandwich complex;
washing the sandwich complex to remove unbound substances;
adding a detection substrate to the washed sandwich complex to detect the concentration of the soluble CD14 subtype in the sample;
where the capture antibody or the detection antibody may be the anti-soluble CD14 subtype monoclonal antibody or the antigen-binding antibody fragment thereof according to the first aspect of the disclosure.

A fourth aspect of the disclosure relates to use of the anti-soluble CD14 subtype monoclonal antibody or the antigen-binding antibody fragment thereof of the first aspect of the disclosure in the preparation of an analysis reagent for identifying whether a patient suffers from sepsis, where the anti-soluble CD14 subtype monoclonal antibody or the antigen-binding antibody fragment thereof of the first aspect of the disclosure is used to detect the concentration of a soluble CD14 subtype in a blood sample from the patient, and the increase in the concentration of the soluble CD14 subtype relative to a reference level is associated with an increase in possibility that the patient suffers from sepsis.

The embodiments of the disclosure also relate to an anti-soluble CD14 subtype monoclonal antibody or an antigen-binding antibody fragment thereof, which specifically recognizes an epitope composed of the amino acid sequence of SEQ ID No. 84, comprising:
(a) VH comprising VH CDR1 composed of the amino acid sequence of SEQ ID NO: 44, VH CDR2 composed of the amino acid sequence of SEQ ID NO: 54, and VH CDR3 composed of the amino acid sequence of SEQ ID NO: 61, and VL comprising VL CDR1 composed of the amino acid sequence of SEQ ID NO: 66, VL CDR2 composed of the amino acid sequence of SEQ ID NO: 75, and VL CDR3 composed of the amino acid sequence of SEQ ID NO: 82;
(b) VH comprising VH CDR1 composed of the amino acid sequence of SEQ ID NO: 45, VH CDR2 composed of the amino acid sequence of SEQ ID NO: 53, and VH CDR3 composed of the amino acid sequence of SEQ ID NO: 57, and VL comprising VL CDR1 composed of the amino acid sequence of SEQ ID NO: 65, VL CDR2 composed of the amino acid sequence of SEQ ID NO: 71, and VL CDR3 composed of the amino acid sequence of SEQ ID NO: 77;
(c) VH comprising VH CDR1 composed of the amino acid sequence of SEQ ID NO: 46, VH CDR2 composed of the amino acid sequence of SEQ ID NO: 52, and VH CDR3 composed of the amino acid sequence of SEQ ID NO: 61, and VL comprising VL CDR1 composed of the amino acid sequence of SEQ ID NO: 68, VL CDR2 composed of the amino acid sequence of SEQ ID NO: 71, and VL CDR3 composed of the amino acid sequence of SEQ ID NO: 83;
(d) VH comprising VH CDR1 composed of the amino acid sequence of SEQ ID NO: 48, VH CDR2 composed of the amino acid sequence of SEQ ID NO: 50, and VH CDR3 composed of the amino acid sequence of SEQ ID NO: 61, and VL comprising VL CDR1 composed of the amino acid sequence of SEQ ID NO: 66, VL CDR2 composed of the amino acid sequence of SEQ ID NO: 70, and VL CDR3 composed of the amino acid sequence of SEQ ID NO: 80;
(e) VH comprising VH CDR1 composed of the amino acid sequence of SEQ ID NO: 48, VH CDR2 composed of the amino acid sequence of SEQ ID NO: 52, and VH CDR3 composed of the amino acid sequence of SEQ ID NO: 61, and VL comprising VL CDR1 composed of the amino acid sequence of SEQ ID NO: 67, VL CDR2 composed of the amino acid sequence of SEQ ID NO: 72, and VL CDR3 composed of the amino acid sequence of SEQ ID NO: 79; or
(f) VH comprising VH CDR1 composed of the amino acid sequence of SEQ ID NO: 49, VH CDR2 composed of the amino acid sequence of SEQ ID NO: 51, and VH CDR3 composed of the amino acid sequence of SEQ ID NO: 62, and VL comprising VL CDR1 composed of the amino acid sequence of SEQ ID NO: 65, VL CDR2 composed of the amino acid sequence of SEQ ID NO: 70, and VL CDR3 composed of the amino acid sequence of SEQ ID NO: 81.

The embodiments of the disclosure further relate to an anti-soluble CD14 subtype monoclonal antibody or an antigen-binding antibody fragment thereof, which specifically recognizes an epitope composed of the amino acid sequence of SEQ ID No. 84, where the soluble CD14 subtype capture antibody is an anti-soluble CD14 subtype monoclonal antibody or an antigen-binding antibody fragment thereof secreted and produced by a hybridoma cell strain with the deposit number of CGMCC NO. 19395.

The anti-soluble CD14 subtype monoclonal antibody and the antigen-binding antibody fragment thereof provided in the embodiments of the disclosure respectively have high affinity for a soluble CD14 subtype, and may thus accurately quantify the concentration of the soluble CD14 subtype in a sample. Moreover, the anti-soluble CD14 subtype monoclonal antibody and the antigen-binding antibody fragment thereof provided in the embodiments of the disclosure respectively have high sensitivity for determination of the concentration of the soluble CD14 subtype. Furthermore, the embodiments of the disclosure may realize high specificity assays in a double-antibody sandwich method, that is, the anti-soluble CD14 subtype monoclonal antibody and the antigen-binding antibody fragment thereof provided in the embodiments of the disclosure only respectively specifically bind to a soluble CD14 subtype present in human blood, but not specifically bind to an sCD14 with a high molecular weight therein.

### BRIEF DESCRIPTION OF THE DRAWINGS

Some embodiments of the disclosure are described below with reference to the accompanying drawings. The objectives and advantages of the disclosure will be further understood from the following detailed description and accompanying drawings. The accompanying drawings show the following:
FIG. 1 shows SDS-PAGE and Western Blot for a prokaryotic expression protein;
FIG. 2 shows SDS-PAGE and Western Blot for a eukaryotic expression protein;
FIG. 3 shows SDS-PAGE and Western Blot for an anti-soluble CD14 subtype polyclonal antibody;
FIG. 4.1 shows a standard curve of kit 1 for detecting a soluble CD14 subtype;
FIG. 4.2 shows a standard curve of kit 2 for detecting a soluble CD14 subtype;
FIG. 4.3 shows a standard curve of kit 3 for detecting a soluble CD14 subtype;
FIG. 4.4 shows a graph of comparison of consistency between kit 1 for detecting a soluble CD14 subtype and kit 3 for detecting a soluble CD14 subtype;
FIG. 4.5 shows a graph of comparison of consistency between kit 1 for detecting a soluble CD14 subtype and kit 2 for detecting a soluble CD14 subtype;
FIG. 5.1 shows a graph of ROC curve of PCT and kit 1 for detecting a soluble CD14 subtype for identifying whether a patient suffers from sepsis;
FIG. 5.2 shows a graph of ROC curve of PCT and kit 2 for detecting a soluble CD14 subtype for identifying whether a patient suffers from sepsis;
FIG. 5.3 shows a graph of comparison of diagnostic power of kit 1, kit 2, and kit 3 for detecting a soluble CD14 subtype.

### DESCRIPTION OF THE PREFERRED EMBODIMENTS

The disclosure will be described below in more detail by way of specific embodiments. However, these embodiments are only representative and the disclosure should in no way be construed as being limited to these embodiments.

In the context of the disclosure, when these expressions, characteristics, values or ranges are referred to in conjunction with expressions such as "about, substantially, generally, at least, at a minimum", the disclosure also includes the exact or precise expressions, characteristics, values or ranges.

In the scope of the disclosure, the "specifically recognizes an epitope composed of the amino acid sequence of SEQ ID No. 84 or SEQ ID No. 42" means that the antibody of the disclosure specifically recognizes a sequence which corresponds to the amino acid sequence of SEQ ID No. 84 or SEQ ID No. 42 in the sequence of a soluble CD14 subtype as an epitope.

In the scope of the disclosure, an antibody sometimes refers to an antibody or an antigen-binding antibody fragment thereof, and an antigen-binding antibody fragment refers to a fragment having the same antigen-binding properties as an original antibody among some of the fragments of an antibody which specifically recognizes an epitope composed of the amino acid sequence of SEQ ID No. 84 or SEQ ID No. 42.

In the scope of the disclosure, unless otherwise stated, a soluble CD14 refers to a human soluble CD14.

In the scope of the disclosure, the term "detection" may be used interchangeably with the terms such as "determination", "quantification" and "analysis", and is meant to encompass both quantitative and qualitative determinations. The detection in the disclosure is preferably performed in vitro.

In the scope of the disclosure, the term ROC (receiver operating characteristic) curve refers to a curve obtained by dividing the diagnostic test results into several critical points, using the sensitivity corresponding to each critical point as the ordinate and the specificity as the abscissa, and plotting. The ROC curve is an effective tool for comprehensive and accurate evaluation of diagnostic tests. Another effect of the ROC curve is to determine the optimal threshold for detection. In most cases when a critical point is determined by an ROC curve method, a point as close to the upper left as possible on the curve is selected, and is determined as the optimal critical point in combination with the professional situation. In the application, according to the ROC curve, combined with the sensitivity and specificity results of each point of tangency, a point of tangency with the largest Youden index as close to the upper left as possible on the curve is selected as the optimal critical point, so that the sensitivity and specificity of the test are high, and the misdiagnosis rate and missed diagnosis rate are low.

The first aspect of the disclosure relates to an anti-soluble CD14 subtype monoclonal antibody or an antigen-binding antibody fragment thereof (also referred to herein as a first monoclonal antibody or an antigen-binding antibody fragment thereof), which specifically recognizes an epitope composed of the amino acid sequence of SEQ ID No. 84, where the anti-soluble CD14 subtype monoclonal antibody or the antigen-binding antibody fragment thereof comprises:
(i) heavy chain variable region (VH) complementarity determining regions (CDRs):
   VH CDR1: X₁X₂X₃MX₄;
   VH CDR2: RIX₅X₆DPX₇; and
   VH CDR3: X₈X₉X₁₀RX₁₁;
      and
(ii) light chain variable region (VL) complementarity determining regions (CDRs):
   VL CDR1: KX₁₂X₁₃X₁₄LA;
   VL CDR2: WX₁₅X₁₆; and
   VL CDR3: KQX₁₇X₁₈;
where X₁ to X₁₈ are one or more of the amino acid sequences listed below as options (see Tables 1 to 6).

**Table 1**

| VH CDR1 | | | | | |
|---|---|---|---|---|---|
| Common sequence | X₁ | X₂ | X₃ | M | X₄ |
| Options | Any one of the amino acids | T or V | A, Y or L | | Q or G |

**Table 2**

| VH CDR2 | | | | | | | |
|---|---|---|---|---|---|---|---|
| Common sequence | R | I | X₅ | X₆ | D | P | X₇ |
| Options | | | TYANG, DPMGG, SPASS, DPANG, or SPANS | GTKA, TTKS, NTKY, TTKS or TTKA | | | KFQA, TKFG, SQFA, MKFG or KFNS |

**Table 3**

| VH CDR3 | | | | | |
|---|---|---|---|---|---|
| Common sequence | X₈ | X₉ | X₁₀ | R | X₁₁ |
| Options | F or G | GG, GQ or CG | YN, YV or KF | | FDY, FYY, FDC or TDC |

**Table 4**

| | VL CDR1 | | | | | |
|---|---|---|---|---|---|---|
| Common sequence | K | X₁₂ | X₁₃ | X₁₄ | L | A |
| Options | | SSAS, STAT, SSQS or STSS | LLKS, LLAS, LLSS or KASS | NRNIHL, RTRKHY, NRKHYL or YTNKAL | | |

**Table 5**

| VL CDR2 | | | |
|---|---|---|---|
| Common sequence | W | X₁₅ | X₁₆ |
| Options | | ATS, AST, QSS or ASK | RAS, LDS, LEA or RES |

**Table 6**

| 'VL CDR3 | | | | |
|---|---|---|---|---|
| Common sequence | K | Q | X₁₇ | X₁₈ |
| Options | | | GYN, YNA, YLN or SYN | LWT, LNT, GNA or TWI |

In some embodiments of the first aspect of the disclosure, the anti-soluble CD14 subtype monoclonal antibody or the antigen-binding antibody fragment thereof comprises VH CDR1, VH CDR2 and VH CDR3, as well as VL CDR1, VL CDR2 and VL CDR3, where VH CDR1, VH CDR2 and VH CDR3, as well as VL CDR1, VL CDR2 and VL CDR3 are selected from the amino acid sequences described in Table 7 (see Table 7).

**Table 7**

| | SEQ ID NO | Amino acid sequences |
|---|---|---|
| VH CDR1 | 44 | DVAMQ |
| | 45 | DTLMQ |
| | 46 | DVAMG |
| | 47 | DTYMG |
| | 48 | DTYMQ |
| | 49 | DVLMG |
| VH CDR2 | 50 | RIDPANGNTKYDPKFQA |
| | 51 | RITYANGGTKADPKFNS |
| | 52 | RIDPMGGNTKYDPKFQA |
| | 53 | RIDPANGNTKYDPSQFA |
| | 54 | RIDPMGGGTKADPKFNS |
| | 55 | RISPANSTTKADPSQFA |
| | 56 | RISPANSTTKADPKFQA |
| VH CDR3 | 57 | FGGYVRFDY |
| | 58 | GCGYVRFYY |
| | 59 | FGGYVRFYY |
| | 60 | GCGYNRFDY |
| | 61 | FGGYNRFDC |
| | 62 | GCGYNRFDC |
| | 63 | GCGKFRTDC |
| VL CDR1 | 64 | KSTSSLLSSNRNIHLLA |
| | 65 | KSSQSLLKSRTRKHYLA |
| | 66 | KSSQSLLSSRTRKHYLA |
| | 67 | KSSASLLKSNRNIHLLA |
| | 68 | KSSASLLSSRTRKHYLA |
| | 69 | KSTSSLLSSNRKHYLLA |
| VL CDR2 | 70 | WASTRES |
| | 71 | WATSRAS |
| | 72 | WQSSRAS |
| | 73 | WATSLDS |
| | 74 | WQSSLEA |
| | 75 | WASKRES |
| VL CDR3 | 76 | KQGYNLWT |
| | 77 | KQGYNLNT |
| | 78 | KQYNALWT |
| | 79 | KQYLNGNA |
| | 80 | KQSYNLWT |
| | 81 | KQYLNLWT |
| | 82 | KQSYNGNA |
| | 83 | KQSYNTWI |

In some embodiments of the first aspect of the disclosure, the anti-soluble CD14 subtype monoclonal antibody or the antigen-binding antibody fragment thereof comprises VH CDR1, VH CDR2 and VH CDR3, as well as VL CDR1, VL CDR2 and VL CDR3, which are any one of the following 1) to 50):
1) VH CDR1 composed of the amino acid sequence of SEQ ID NO: 44, VH CDR2 composed of the amino acid sequence of SEQ ID NO: 50, VH CDR3 composed of the amino acid sequence of SEQ ID NO: 61, VL CDR1 composed of the amino acid sequence of SEQ ID NO: 69, VL CDR2 composed of the amino acid sequence of SEQ ID NO: 75, and VL CDR3 composed of the amino acid sequence of SEQ ID NO: 76;
2) VH CDR1 composed of the amino acid sequence of SEQ ID NO: 44, VH CDR2 composed of the amino acid sequence of SEQ ID NO: 51, VH CDR3 composed of the amino acid sequence of SEQ ID NO: 57, VL CDR1 composed of the amino acid sequence of SEQ ID NO: 64, VL CDR2 composed of the amino acid sequence of SEQ ID NO: 73, and VL CDR3 composed of the amino acid sequence of SEQ ID NO: 78;
3) VH CDR1 composed of the amino acid sequence of SEQ ID NO: 44, VH CDR2 composed of the amino acid sequence of SEQ ID NO: 53, VH CDR3 composed of the amino acid sequence of SEQ ID NO: 59, VL CDR1 composed of the amino acid sequence of SEQ ID NO: 67, VL CDR2 composed of the amino acid sequence of SEQ ID NO: 71, and VL CDR3 composed of the amino acid sequence of SEQ ID NO: 80;
4) VH CDR1 composed of the amino acid sequence of SEQ ID NO: 44, VH CDR2 composed of the amino acid sequence of SEQ ID NO: 53, VH CDR3 composed of the amino acid sequence of SEQ ID NO: 57, VL CDR1 composed of the amino acid sequence of SEQ ID NO: 68, VL CDR2 composed of the amino acid sequence of SEQ ID NO: 73, and VL CDR3 composed of the amino acid sequence of SEQ ID NO: 79;
5) VH CDR1 composed of the amino acid sequence of SEQ ID NO: 44, VH CDR2 composed of the amino acid sequence of SEQ ID NO: 54, VH CDR3 composed of the amino acid sequence of SEQ ID NO: 61, VL CDR1 composed of the amino acid sequence of SEQ ID NO: 66, VL CDR2 composed of the amino acid sequence of SEQ ID NO: 75, and VL CDR3 composed of the amino acid sequence of SEQ ID NO: 82;
6) VH CDR1 composed of the amino acid sequence of SEQ ID NO: 44, VH CDR2 composed of the amino acid sequence of SEQ ID NO: 54, VH CDR3 composed of the amino acid sequence of SEQ ID NO: 57, VL CDR1 composed of the amino acid sequence of SEQ ID NO: 64, VL CDR2 composed of the amino acid sequence of SEQ ID NO: 75, and VL CDR3 composed of the amino acid sequence of SEQ ID NO: 76;
7) VH CDR1 composed of the amino acid sequence of SEQ ID NO: 44, VH CDR2 composed of the amino acid sequence of SEQ ID NO: 56, VH CDR3 composed of the amino acid sequence of SEQ ID NO: 58, VL CDR1 composed of the amino acid sequence of SEQ ID NO: 68, VL CDR2 composed of the amino acid sequence of SEQ ID NO: 74, and VL CDR3 composed of the amino acid sequence of SEQ ID NO: 80;
8) VH CDR1 composed of the amino acid sequence of SEQ ID NO: 44, VH CDR2 composed of the amino acid sequence of SEQ ID NO: 56, VH CDR3 composed of the amino acid sequence of SEQ ID NO: 59, VL CDR1 composed of the amino acid sequence of SEQ ID NO: 66, VL CDR2 composed of the amino acid sequence of SEQ ID NO: 71, and VL CDR3 composed of the amino acid sequence of SEQ ID NO: 80;
9) VH CDR1 composed of the amino acid sequence of SEQ ID NO: 45, VH CDR2 composed of the amino acid sequence of SEQ ID NO: 50, VH CDR3 composed of the amino acid sequence of SEQ ID NO: 59, VL CDR1 composed of the amino acid sequence of SEQ ID NO: 64, VL CDR2 composed of the amino acid sequence of SEQ ID NO: 75, and VL CDR3 composed of the amino acid sequence of SEQ ID NO: 76;
10) VH CDR1 composed of the amino acid sequence of SEQ ID NO: 45, VH CDR2 composed of the amino acid sequence of SEQ ID NO: 51, VH CDR3 composed of the amino acid sequence of SEQ ID NO: 58, VL CDR1 composed of the amino acid sequence of SEQ ID NO: 66, VL CDR2 composed of the amino acid sequence of SEQ ID NO: 71, and VL CDR3 composed of the amino acid sequence of SEQ ID NO: 80;
11) VH CDR1 composed of the amino acid sequence of SEQ ID NO: 45, VH CDR2 composed of the amino acid sequence of SEQ ID NO: 51, VH CDR3 composed of the amino acid sequence of SEQ ID NO: 60, VL CDR1 composed of the amino acid sequence of SEQ ID NO: 67, VL CDR2 composed of the amino acid sequence of SEQ ID NO: 70, and VL CDR3 composed of the amino acid sequence of SEQ ID NO: 79;
12) VH CDR1 composed of the amino acid sequence of SEQ ID NO: 45, VH CDR2 composed of the amino acid sequence of SEQ ID NO: 52, VH CDR3 composed of the amino acid sequence of SEQ ID NO: 60, VL CDR1 composed of the amino acid sequence of SEQ ID NO: 66, VL CDR2 composed of the amino acid sequence of SEQ ID NO: 70, and VL CDR3 composed of the amino acid sequence of SEQ ID NO: 76;
13) VH CDR1 composed of the amino acid sequence of SEQ ID NO: 45, VH CDR2 composed of the amino acid sequence of SEQ ID NO: 53, VH CDR3 composed of the amino acid sequence of SEQ ID NO: 58, VL CDR1 composed of the amino acid sequence of SEQ ID NO: 69, VL CDR2 composed of the amino acid sequence of SEQ ID NO: 73, and VL CDR3 composed of the amino acid sequence of SEQ ID NO: 82;
14) VH CDR1 composed of the amino acid sequence of SEQ ID NO: 45, VH CDR2 composed of the amino acid sequence of SEQ ID NO: 53, VH CDR3 composed of the amino acid sequence of SEQ ID NO: 57, VL CDR1 composed of the amino acid sequence of SEQ ID NO: 65, VL CDR2 composed of the amino acid sequence of SEQ ID NO: 71, and VL CDR3 composed of the amino acid sequence of SEQ ID NO: 77;
15) VH CDR1 composed of the amino acid sequence of SEQ ID NO: 45, VH CDR2 composed of the amino acid sequence of SEQ ID NO: 54, VH CDR3 composed of the amino acid sequence of SEQ ID NO: 59, VL CDR1 composed of the amino acid sequence of SEQ ID NO: 66, VL CDR2 composed of the amino acid sequence of SEQ ID NO: 75, and VL CDR3 composed of the amino acid sequence of SEQ ID NO: 81;
16) VH CDR1 composed of the amino acid sequence of SEQ ID NO: 45, VH CDR2 composed of the amino acid sequence of SEQ ID NO: 55, VH CDR3 composed of the amino acid sequence of SEQ ID NO: 57, VL CDR1 composed of the amino acid sequence of SEQ ID NO: 68, VL CDR2 composed of the amino acid sequence of SEQ ID NO: 73, and VL CDR3 composed of the amino acid sequence of SEQ ID NO: 78;
17) VH CDR1 composed of the amino acid sequence of SEQ ID NO: 45, VH CDR2 composed of the amino acid sequence of SEQ ID NO: 56, VH CDR3 composed of the amino acid sequence of SEQ ID NO: 62, VL CDR1 composed of the amino acid sequence of SEQ ID NO: 69, VL CDR2 composed of the amino acid sequence of SEQ ID NO: 71, and VL CDR3 composed of the amino acid sequence of SEQ ID NO: 78;
18) VH CDR1 composed of the amino acid sequence of SEQ ID NO: 45, VH CDR2 composed of the amino acid sequence of SEQ ID NO: 56, VH CDR3 composed of the amino acid sequence of SEQ ID NO: 59, VL CDR1 composed of the amino acid sequence of SEQ ID NO: 69, VL CDR2 composed of the amino acid sequence of SEQ ID NO: 71, and VL CDR3 composed of the amino acid sequence of SEQ ID NO: 83;
19) VH CDR1 composed of the amino acid sequence of SEQ ID NO: 46, VH CDR2 composed of the amino acid sequence of SEQ ID NO: 50, VH CDR3 composed of the amino acid sequence of SEQ ID NO: 62, VL CDR1 composed of the amino acid sequence of SEQ ID NO: 69, VL CDR2 composed of the amino acid sequence of SEQ ID NO: 70, and VL CDR3 composed of the amino acid sequence of SEQ ID NO: 81;
20) VH CDR1 composed of the amino acid sequence of SEQ ID NO: 46, VH CDR2 composed of the amino acid sequence of SEQ ID NO: 51, VH CDR3 composed of the amino acid sequence of SEQ ID NO: 58, VL CDR1 composed of the amino acid sequence of SEQ ID NO: 67, VL CDR2 composed of the amino acid sequence of SEQ ID NO: 73, and VL CDR3 composed of the amino acid sequence of SEQ ID NO: 83;
21) VH CDR1 composed of the amino acid sequence of SEQ ID NO: 46, VH CDR2 composed of the amino acid sequence of SEQ ID NO: 52, VH CDR3 composed of the amino acid sequence of SEQ ID NO: 61, VL CDR1 composed of the amino acid sequence of SEQ ID NO: 68, VL CDR2 composed of the amino acid sequence of SEQ ID NO: 71, and VL CDR3 composed of the amino acid sequence of SEQ ID NO: 83;
22) VH CDR1 composed of the amino acid sequence of SEQ ID NO: 46, VH CDR2 composed of the amino acid sequence of SEQ ID NO: 52, VH CDR3 composed of the amino acid sequence of SEQ ID NO: 60, VL CDR1 composed of the amino acid sequence of SEQ ID NO: 67, VL CDR2 composed of the amino acid sequence of SEQ ID NO: 74, and VL CDR3 composed of the amino acid sequence of SEQ ID NO: 81;
23) VH CDR1 composed of the amino acid sequence of SEQ ID NO: 46, VH CDR2 composed of the amino acid sequence of SEQ ID NO: 52, VH CDR3 composed of the amino acid sequence of SEQ ID NO: 59, VL CDR1 composed of the amino acid sequence of SEQ ID NO: 69, VL CDR2 composed of the amino acid sequence of SEQ ID NO: 75, and VL CDR3 composed of the amino acid sequence of SEQ ID NO: 83;
24) VH CDR1 composed of the amino acid sequence of SEQ ID NO: 46, VH CDR2 composed of the amino acid sequence of SEQ ID NO: 53, VH CDR3 composed of the amino acid sequence of SEQ ID NO: 60, VL CDR1 composed of the amino acid sequence of SEQ ID NO: 64, VL CDR2 composed of the amino acid sequence of SEQ ID NO: 72, and VL CDR3 composed of the amino acid sequence of SEQ ID NO: 76;
25) VH CDR1 composed of the amino acid sequence of SEQ ID NO: 46, VH CDR2 composed of the amino acid sequence of SEQ ID NO: 54, VH CDR3 composed of the amino acid sequence of SEQ ID NO: 63, VL CDR1 composed of the amino acid sequence of SEQ ID NO: 66, VL CDR2 composed of the amino acid sequence of SEQ ID NO: 71, and VL CDR3 composed of the amino acid sequence of SEQ ID NO: 79;
26) VH CDR1 composed of the amino acid sequence of SEQ ID NO: 46, VH CDR2 composed of the amino acid sequence of SEQ ID NO: 55, VH CDR3 composed of the amino acid sequence of SEQ ID NO: 57, VL CDR1 composed of the amino acid sequence of SEQ ID NO: 64, VL CDR2 composed of the amino acid sequence of SEQ ID NO: 75, and VL CDR3 composed of the amino acid sequence of SEQ ID NO: 77;
27) VH CDR1 composed of the amino acid sequence of SEQ ID NO: 46, VH CDR2 composed of the amino acid sequence of SEQ ID NO: 56, VH CDR3 composed of the amino acid sequence of SEQ ID NO: 63, VL CDR1 composed of the amino acid sequence of SEQ ID NO: 65, VL CDR2 composed of the amino acid sequence of SEQ ID NO: 73, and VL CDR3 composed of the amino acid sequence of SEQ ID NO: 79;
28) VH CDR1 composed of the amino acid sequence of SEQ ID NO: 46, VH CDR2 composed of the amino acid sequence of SEQ ID NO: 56, VH CDR3 composed of the amino acid sequence of SEQ ID NO: 58, VL CDR1 composed of the amino acid sequence of SEQ ID NO: 65, VL CDR2 composed of the amino acid sequence of SEQ ID NO: 75, and VL CDR3 composed of the amino acid sequence of SEQ ID NO: 82;
29) VH CDR1 composed of the amino acid sequence of SEQ ID NO: 47, VH CDR2 composed of the amino acid sequence of SEQ ID NO: 51, VH CDR3 composed of the amino acid sequence of SEQ ID NO: 57, VL CDR1 composed of the amino acid sequence of SEQ ID NO: 65, VL CDR2 composed of the amino acid sequence of SEQ ID NO: 72, and VL CDR3 composed of the amino acid sequence of SEQ ID NO: 77;
30) VH CDR1 composed of the amino acid sequence of SEQ ID NO: 47, VH CDR2 composed of the amino acid sequence of SEQ ID NO: 52, VH CDR3 composed of the amino acid sequence of SEQ ID NO: 62, VL CDR1 composed of the amino acid sequence of SEQ ID NO: 65, VL CDR2 composed of the amino acid sequence of SEQ ID NO: 74, and VL CDR3 composed of the amino acid sequence of SEQ ID NO: 77;
31) VH CDR1 composed of the amino acid sequence of SEQ ID NO: 47, VH CDR2 composed of the amino acid sequence of SEQ ID NO: 53, VH CDR3 composed of the amino acid sequence of SEQ ID NO: 61, VL CDR1 composed of the amino acid sequence of SEQ ID NO: 68, VL CDR2 composed of the amino acid sequence of SEQ ID NO: 72, and VL CDR3 composed of the amino acid sequence of SEQ ID NO: 77;
32) VH CDR1 composed of the amino acid sequence of SEQ ID NO: 47, VH CDR2 composed of the amino acid sequence of SEQ ID NO: 53, VH CDR3 composed of the amino acid sequence of SEQ ID NO: 57, VL CDR1 composed of the amino acid sequence of SEQ ID NO: 67, VL CDR2 composed of the amino acid sequence of SEQ ID NO: 71, and VL CDR3 composed of the amino acid sequence of SEQ ID NO: 77;
33) VH CDR1 composed of the amino acid sequence of SEQ ID NO: 47, VH CDR2 composed of the amino acid sequence of SEQ ID NO: 55, VH CDR3 composed of the amino acid sequence of SEQ ID NO: 60, VL CDR1 composed of the amino acid sequence of SEQ ID NO: 69, VL CDR2 composed of the amino acid sequence of SEQ ID NO: 75, and VL CDR3 composed of the amino acid sequence of SEQ ID NO: 83;
34) VH CDR1 composed of the amino acid sequence of SEQ ID NO: 47, VH CDR2 composed of the amino acid sequence of SEQ ID NO: 55, VH CDR3 composed of the amino acid sequence of SEQ ID NO: 58, VL CDR1 composed of the amino acid sequence of SEQ ID NO: 64, VL CDR2 composed of the amino acid sequence of SEQ ID NO: 73, and VL CDR3 composed of the amino acid sequence of SEQ ID NO: 83;
35) VH CDR1 composed of the amino acid sequence of SEQ ID NO: 47, VH CDR2 composed of the amino acid sequence of SEQ ID NO: 56, VH CDR3 composed of the amino acid sequence of SEQ ID NO: 63, VL CDR1 composed of the amino acid sequence of SEQ ID NO: 65, VL CDR2 composed of the amino acid sequence of SEQ ID NO: 70, and VL CDR3 composed of the amino acid sequence of SEQ ID NO: 82;
36) VH CDR1 composed of the amino acid sequence of SEQ ID NO: 48, VH CDR2 composed of the amino acid sequence of SEQ ID NO: 50, VH CDR3 composed of the amino acid sequence of SEQ ID NO: 61, VL CDR1 composed of the amino acid sequence of SEQ ID NO: 66, VL CDR2 composed of the amino acid sequence of SEQ ID NO: 70, and VL CDR3 composed of the amino acid sequence of SEQ ID NO: 80;
37) VH CDR1 composed of the amino acid sequence of SEQ ID NO: 48, VH CDR2 composed of the amino acid sequence of SEQ ID NO: 50, VH CDR3 composed of the amino acid sequence of SEQ ID NO: 58, VL CDR1 composed of the amino acid sequence of SEQ ID NO: 66, VL CDR2 composed of the amino acid sequence of SEQ ID NO: 70, and VL CDR3 composed of the amino acid sequence of SEQ ID NO: 82;
38) VH CDR1 composed of the amino acid sequence of SEQ ID NO: 48, VH CDR2 composed of the amino acid sequence of SEQ ID NO: 51, VH CDR3 composed of the amino acid sequence of SEQ ID NO: 62, VL CDR1 composed of the amino acid sequence of SEQ ID NO: 64, VL CDR2 composed of the amino acid sequence of SEQ ID NO: 75, and VL CDR3 composed of the amino acid sequence of SEQ ID NO: 81;
39) VH CDR1 composed of the amino acid sequence of SEQ ID NO: 48, VH CDR2 composed of the amino acid sequence of SEQ ID NO: 52, VH CDR3 composed of the amino acid sequence of SEQ ID NO: 61, VL CDR1 composed of the amino acid sequence of SEQ ID NO: 67, VL CDR2 composed of the amino acid sequence of SEQ ID NO: 72, and VL CDR3 composed of the amino acid sequence of SEQ ID NO: 79;
40) VH CDR1 composed of the amino acid sequence of SEQ ID NO: 48, VH CDR2 composed of the amino acid sequence of SEQ ID NO: 52, VH CDR3 composed of the amino acid sequence of SEQ ID NO: 59, VL CDR1 composed of the amino acid sequence of SEQ ID NO: 67, VL CDR2 composed of the amino acid sequence of SEQ ID NO: 72, and VL CDR3 composed of the amino acid sequence of SEQ ID NO: 80;
41) VH CDR1 composed of the amino acid sequence of SEQ ID NO: 48, VH CDR2 composed of the amino acid sequence of SEQ ID NO: 53, VH CDR3 composed of the amino acid sequence of SEQ ID NO: 61, VL CDR1 composed of the amino acid sequence of SEQ ID NO: 65, VL CDR2 composed of the amino acid sequence of SEQ ID NO: 71, and VL CDR3 composed of the amino acid sequence of SEQ ID NO: 80;
42) VH CDR1 composed of the amino acid sequence of SEQ ID NO: 48, VH CDR2 composed of the amino acid sequence of SEQ ID NO: 54, VH CDR3 composed of the amino acid sequence of SEQ ID NO: 58, VL CDR1 composed of the amino acid sequence of SEQ ID NO: 66, VL CDR2 composed of the amino acid sequence of SEQ ID NO: 71, and VL CDR3 composed of the amino acid sequence of SEQ ID NO: 82;
43) VH CDR1 composed of the amino acid sequence of SEQ ID NO: 48, VH CDR2 composed of the amino acid sequence of SEQ ID NO: 54, VH CDR3 composed of the amino acid sequence of SEQ ID NO: 57, VL CDR1 composed of the amino acid sequence of SEQ ID NO: 64, VL CDR2 composed of the amino acid sequence of SEQ ID NO: 72, and VL CDR3 composed of the amino acid sequence of SEQ ID NO: 77;
44) VH CDR1 composed of the amino acid sequence of SEQ ID NO: 48, VH CDR2 composed of the amino acid sequence of SEQ ID NO: 55, VH CDR3 composed of the amino acid sequence of SEQ ID NO: 58, VL CDR1 composed of the amino acid sequence of SEQ ID NO: 69, VL CDR2 composed of the amino acid sequence of SEQ ID NO: 75, and VL CDR3 composed of the amino acid sequence of SEQ ID NO: 81;
45) VH CDR1 composed of the amino acid sequence of SEQ ID NO: 48, VH CDR2 composed of the amino acid sequence of SEQ ID NO: 55, VH CDR3 composed of the amino acid sequence of SEQ ID NO: 63, VL CDR1 composed of the amino acid sequence of SEQ ID NO: 64, VL CDR2 composed of the amino acid sequence of SEQ ID NO: 70, and VL CDR3 composed of the amino acid sequence of SEQ ID NO: 76;
46) VH CDR1 composed of the amino acid sequence of SEQ ID NO: 49, VH CDR2 composed of the amino acid sequence of SEQ ID NO: 50, VH CDR3 composed of the amino acid sequence of SEQ ID NO: 60, VL CDR1 composed of the amino acid sequence of SEQ ID NO: 66, VL CDR2 composed of the amino acid sequence of SEQ ID NO: 72, and VL CDR3 composed of the amino acid sequence of SEQ ID NO: 81;
47) VH CDR1 composed of the amino acid sequence of SEQ ID NO: 49, VH CDR2 composed of the amino acid sequence of SEQ ID NO: 51, VH CDR3 composed of the amino acid sequence of SEQ ID NO: 60, VL CDR1 composed of the amino acid sequence of SEQ ID NO: 66, VL CDR2 composed of the amino acid sequence of SEQ ID NO: 74, and VL CDR3 composed of the amino acid sequence of SEQ ID NO: 83;
48) VH CDR1 composed of the amino acid sequence of SEQ ID NO: 49, VH CDR2 composed of the amino acid sequence of SEQ ID NO: 51, VH CDR3 composed of the amino acid sequence of SEQ ID NO: 62, VL CDR1 composed of the amino acid sequence of SEQ ID NO: 65, VL CDR2 composed of the amino acid sequence of SEQ ID NO: 70, and VL CDR3 composed of the amino acid sequence of SEQ ID NO: 81;
49) VH CDR1 composed of the amino acid sequence of SEQ ID NO: 49, VH CDR2 composed of the amino acid sequence of SEQ ID NO: 52, VH CDR3 composed of the amino acid sequence of SEQ ID NO: 59, VL CDR1 composed of the amino acid sequence of SEQ ID NO: 66, VL CDR2 composed of the amino acid sequence of SEQ ID NO: 70, and VL CDR3 composed of the amino acid sequence of SEQ ID NO: 79; or
50) VH CDR1 composed of the amino acid sequence of SEQ ID NO: 49, VH CDR2 composed of the amino acid sequence of SEQ ID NO: 53, VH CDR3 composed of the amino acid sequence of SEQ ID NO: 60, VL CDR1 composed of the amino acid sequence of SEQ ID NO: 68, VL CDR2 composed of the amino acid sequence of SEQ ID NO: 71, and VL CDR3 composed of the amino acid sequence of SEQ ID NO: 77.

Some embodiments of the first aspect of the disclosure relate to an anti-soluble CD14 subtype monoclonal antibody or an antigen-binding antibody fragment thereof, which specifically recognizes an epitope composed of the amino acid sequence of SEQ ID No. 84, comprising:
(a) VH comprising VH CDR1 composed of the amino acid sequence of SEQ ID NO: 44, VH CDR2 composed of the amino acid sequence of SEQ ID NO: 54, and VH CDR3 composed of the amino acid sequence of SEQ ID NO: 61, and VL comprising VL CDR1 composed of the amino acid sequence of SEQ ID NO: 66, VL CDR2 composed of the amino acid sequence of SEQ ID NO: 75, and VL CDR3 composed of the amino acid sequence of SEQ ID NO: 82;
(b) VH comprising VH CDR1 composed of the amino acid sequence of SEQ ID NO: 45, VH CDR2 composed of the amino acid sequence of SEQ ID NO: 53, and VH CDR3 composed of the amino acid sequence of SEQ ID NO: 57, and VL comprising VL CDR1 composed of the amino acid sequence of SEQ ID NO: 65, VL CDR2 composed of the amino acid sequence of SEQ ID NO: 71, and VL CDR3 composed of the amino acid sequence of SEQ ID NO: 77;
(c) VH comprising VH CDR1 composed of the amino acid sequence of SEQ ID NO: 46, VH CDR2 composed of the amino acid sequence of SEQ ID NO: 52, and VH CDR3 composed of the amino acid sequence of SEQ ID NO: 61, and VL comprising VL CDR1 composed of the amino acid sequence of SEQ ID NO: 68, VL CDR2 composed of the amino acid sequence of SEQ ID NO: 71, and VL CDR3 composed of the amino acid sequence of SEQ ID NO: 83;
(d) VH comprising VH CDR1 composed of the amino acid sequence of SEQ ID NO: 48, VH CDR2 composed of the amino acid sequence of SEQ ID NO: 50, and VH CDR3 composed of the amino acid sequence of SEQ ID NO: 61, and VL comprising VL CDR1 composed of the amino acid sequence of SEQ ID NO: 66, VL CDR2 composed of the amino acid sequence of SEQ ID NO: 70, and VL CDR3 composed of the amino acid sequence of SEQ ID NO: 80;
(e) VH comprising VH CDR1 composed of the amino acid sequence of SEQ ID NO: 48, VH CDR2 composed of the amino acid sequence of SEQ ID NO: 52, and VH CDR3 composed of the amino acid sequence of SEQ ID NO: 61, and VL comprising VL CDR1 composed of the amino acid sequence of SEQ ID NO: 67, VL CDR2 composed of the amino acid sequence of SEQ ID NO: 72, and VL CDR3 composed of the amino acid sequence of SEQ ID NO: 79; or
(f) VH comprising VH CDR1 composed of the amino acid sequence of SEQ ID NO: 49, VH CDR2 composed of the amino acid sequence of SEQ ID NO: 51, and VH CDR3 composed of the amino acid sequence of SEQ ID NO: 62, and VL comprising VL CDR1 composed of the amino acid sequence of SEQ ID NO: 65, VL CDR2 composed of the amino acid sequence of SEQ ID NO: 70, and VL CDR3 composed of the amino acid sequence of SEQ ID NO: 81.

In some embodiments of the first aspect of the disclosure, the anti-soluble CD14 monoclonal antibody or the antigen-binding antibody fragment thereof is an anti-soluble CD14 subtype monoclonal antibody or an antigen-binding antibody fragment thereof secreted and produced by a hybridoma cell strain with the deposit number of CGMCC NO. 19395.

In some embodiments of the first aspect of the disclosure, the anti-soluble CD14 subtype monoclonal antibody or the antigen-binding antibody fragment thereof does not specifically bind to a soluble CD14 with a high molecular weight.

In some embodiments of the first aspect of the disclosure, the antigen-binding antibody fragment may be an antigen-binding antibody fragment selected from a group consisting of Fab, Fab', F(ab')2, a single-chain antibody (scFV), a dimerized V region (a dimeric antibody), a disulfide stabilized V region (dsFv), sc(Fv)2, a CDR-containing polypeptide, a heavy chain variable region-containing polypeptide, and a light chain variable region-containing polypeptide.

The second aspect of the disclosure relates to a kit for detecting a soluble CD14 subtype, where the kit for detecting a soluble CD14 subtype comprises at least the first monoclonal antibody or the antigen-binding antibody fragment thereof according to the disclosure.

Preferably, the kit for detecting a soluble CD14 subtype according to the second aspect of the disclosure further comprises another anti-soluble CD14 subtype monoclonal antibody or an antigen-binding antibody fragment thereof (also referred to herein as a second monoclonal antibody or an antigen-binding antibody fragment thereof).

In some embodiments of the second aspect of the disclosure, the second monoclonal antibody or the antigen-binding antibody fragment thereof specifically recognizes an epitope composed of the amino acid sequence of SEQ ID No. 42, where the anti-soluble CD14 subtype monoclonal antibody or the antigen-binding antibody fragment thereof comprises:
(i) heavy chain variable region (VH) complementarity determining regions (CDRs):
   VH CDR1: X₁X₂X₃MX₄;
   VH CDR2: YIX₅X₆ADX₇; and
   VH CDR3: X₈X₉X₁₀AX₁₁;
      and
(ii) light chain variable region (VL) complementarity determining regions (CDRs):
   VL CDR1: KX₁₂X₁₃X₁₄N;
   VL CDR2: LX₁₅X₁₆; and
   VL CDR3: VX₁₇X₁₈X₁₉;
where X₁ to X₁₉ are one or more of the amino acid sequences listed below as options (see Tables 8 to 13).

**Table 8**

| VH CDR1 | | | | | |
|---|---|---|---|---|---|
| Common sequence | X₁ | X₂ | X₃ | M | X₄ |
| Options | Any one of the amino acids | F or V | A, E or K | | A or L |

**Table 9**

| VH CDR2 | | | | | | | |
|---|---|---|---|---|---|---|---|
| Common sequence | Y | I | X₅ | X₆ | A | D | X₇ |
| Options | | | SYMGS, SSGSS, AYTMY, TYSGS or SSKSS | GAYY, TIYY, AKYY, TKAS or SNLA | | | AKLG, TVKG, SYTA, MTKG or YGNT |

**Table 10**

| VH CDR3 | | | | | |
|---|---|---|---|---|---|
| Common sequence | X₈ | X₉ | X₁₀ | A | X₁₁ |
| Options | A or Q | GorQ | Q or F | | M, Y or V |

**Table 11**

| VL CDR1 | | | | | |
|---|---|---|---|---|---|
| Common sequence | K | X₁₂ | X₁₃ | X₁₄ | N |
| Options | | YYAS, SSAT, SSQS or SGSS | AAKL, LLAT, LLYS or KAAS | YRNIKL, TWAKGN, NGKTYL or YTNGAL | |

**Table 12**

| VL CDR2 | | | |
|---|---|---|---|
| Common sequence | L | X₁₅ | X₁₆ |
| Options | | VQS, KTS, QTS or VSK | LAS, LDS, LTA or DSK |

**Table 13**

| VL CDR3 | | | | |
|---|---|---|---|---|
| Common sequence | V | X₁₇ | X₁₈ | X₁₉ |
| Options | | G, A, S or Q | GTH, GNT, GTA or TAI | FITA, FPRT, YGHV or NYGH |

In some embodiments of the second monoclonal antibody or the antigen-binding antibody fragment thereof of the disclosure, the second monoclonal antibody or the antigen-binding antibody fragment thereof comprises VH CDR1, VH CDR2 and VH CDR3, as well as VL CDR1, VL CDR2 and VL CDR3, where VH CDR1, VH CDR2 and VH CDR3, as well as VL CDR1, VL CDR2 and VL CDR3 are selected from the amino acid sequences described in Table 14 (see Table 14).

**Table 14**

| | SEQ ID NO | | Amino acid sequences |
|---|---|---|---|
| VH CDR1 | | 1 | SVAML |
| | | 2 | SVEML |
| | | 3 | SFAML |
| | | 4 | SFAMA |
| | | 5 | SVKML |
| | | 6 | SFKMA |
| VH CDR2 | | 7 | YISYMGSTIYYADAKLG |
| | | 8 | YISSKSSSNLAADAKLG |
| | | 9 | YISSGSSTIYYADTVKG |
| | | 10 | YISSGSSTIYYADMTKG |
| | | 11 | YITYSGSGAYYADYGNT |
| | | 12 | YISYMGSTIYYADTVKG |
| | | 13 | YISSKSSTIYYADTVKG |
| VH CDR3 | | 14 | AGFAY |
| | | 15 | QQFAY |
| | | 16 | QGFAY |
| | | 17 | QGFAM |
| | | 18 | QQVAY |
| | | 19 | AGFAM |
| | | 20 | AGFAY |
| VL CDR1 | | 21 | KSSQSLLYSNGKTYLN |
| | | 22 | KYYASAAKLYRNIKLN |
| | | 23 | KYYASLLATYRNIKLN |
| | | 24 | KYYASLLATTWAKGNN |
| | | 25 | KSSQSLLYSTWAKGNN |
| | | 26 | KSSQSLLYSYRNIKLN |
| VL CDR2 | | 27 | LVQSLDS |
| | | 28 | LVSKLTA |
| | | 29 | LVSKLDS |
| | | 30 | LVQSDSK |
| | | 31 | LQTSDSK |
| | | 32 | LVSKLAS |
| VL CDR3 | | 33 | VQGTHNYGH |
| | | 34 | VGGTHFPRT |
| | | 35 | VGTAIFPRT |
| | | 36 | VGGTHFITA |
| | | 37 | VSGTHFPRT |
| | | 38 | VQGTHFPRT |
| | | 39 | VQTAIFPRT |
| | | 40 | VQGTHYGHV |
| | | 41 | VQGNTFITA |

In some embodiments of the second monoclonal antibody or the antigen-binding antibody fragment thereof of the disclosure, the second monoclonal antibody or the antigen-binding antibody fragment thereof comprises VH CDR1, VH CDR2 and VH CDR3, as well as VL CDR1, VL CDR2 and VL CDR3, where VH CDR1, VH CDR2 and VH CDR3, as well as VL CDR1, VL CDR2 and VL CDR3 are selected from the amino acid sequences described in Table 15 (see Table 15).

**Table 15**

| | SEQ ID NO | | Amino acid sequences |
|---|---|---|---|
| VH CDR1 | | 1 | SVAML |
| | | 2 | SVEML |
| | | 3 | SFAML |
| | | 4 | SFAMA |
| | | 5 | SVKML |
| | | 6 | SFKMA |
| VH CDR2 | | 7 | YISYMGSTIYYADAKLG |
| | | 8 | YISSKSSSNLAADAKLG |
| | | 9 | YISSGSSTIYYADTVKG |
| | | 10 | YISSGSSTIYYADMTKG |
| VH CDR3 | | 14 | AGFAY |
| | | 15 | QQFAY |
| | | 16 | QGFAY |
| | | 17 | QGFAM |
| VL CDR1 | | 21 | KSSQSLLYSNGKTYLN |
| | | 22 | KYYASAAKLYRNIKLN |
| | | 23 | KYYASLLATYRNIKLN |
| | | 24 | KYYASLLATTWAKGNN |
| VL CDR2 | | 27 | LVQSLDS |
| | | 28 | LVSKLTA |
| | | 29 | LVSKLDS |
| | | 30 | LVQSDSK |
| VL CDR3 | | 33 | VQGTHNYGH |
| | | 34 | VGGTHFPRT |
| | | 35 | VGTAIFPRT |
| | | 36 | VGGTHFITA |
| | | 38 | VQGTHFPRT |

In some embodiments of the second monoclonal antibody or the antigen-binding antibody fragment thereof of the disclosure, the second monoclonal antibody or the antigen-binding antibody fragment thereof comprises VH CDR1, VH CDR2 and VH CDR3, as well as VL CDR1, VL CDR2 and VL CDR3, which are any one of the following 1) to 62):
1) VH CDR1 composed of the amino acid sequence of SEQ ID NO: 1, VH CDR2 composed of the amino acid sequence of SEQ ID NO: 10, VH CDR3 composed of the amino acid sequence of SEQ ID NO: 14, VL CDR1 composed of the amino acid sequence of SEQ ID NO: 21, VL CDR2 composed of the amino acid sequence of SEQ ID NO: 32, and VL CDR3 composed of the amino acid sequence of SEQ ID NO: 34;
2) VH CDR1 composed of the amino acid sequence of SEQ ID NO: 1, VH CDR2 composed of the amino acid sequence of SEQ ID NO: 11, VH CDR3 composed of the amino acid sequence of SEQ ID NO: 17, VL CDR1 composed of the amino acid sequence of SEQ ID NO: 26, VL CDR2 composed of the amino acid sequence of SEQ ID NO: 28, and VL CDR3 composed of the amino acid sequence of SEQ ID NO: 41;
3) VH CDR1 composed of the amino acid sequence of SEQ ID NO: 1, VH CDR2 composed of the amino acid sequence of SEQ ID NO: 12, VH CDR3 composed of the amino acid sequence of SEQ ID NO: 17, VL CDR1 composed of the amino acid sequence of SEQ ID NO: 21, VL CDR2 composed of the amino acid sequence of SEQ ID NO: 32, and VL CDR3 composed of the amino acid sequence of SEQ ID NO: 33;
4) VH CDR1 composed of the amino acid sequence of SEQ ID NO: 1, VH CDR2 composed of the amino acid sequence of SEQ ID NO: 12, VH CDR3 composed of the amino acid sequence of SEQ ID NO: 14, VL CDR1 composed of the amino acid sequence of SEQ ID NO: 26, VL CDR2 composed of the amino acid sequence of SEQ ID NO: 32, and VL CDR3 composed of the amino acid sequence of SEQ ID NO: 39;
5) VH CDR1 composed of the amino acid sequence of SEQ ID NO: 1, VH CDR2 composed of the amino acid sequence of SEQ ID NO: 13, VH CDR3 composed of the amino acid sequence of SEQ ID NO: 20, VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 40;
6) VH CDR1 composed of the amino acid sequence of SEQ ID NO: 2, VH CDR2 composed of the amino acid sequence of SEQ ID NO: 7, VH CDR3 composed of the amino acid sequence of SEQ ID NO: 17, VL CDR1 composed of the amino acid sequence of SEQ ID NO: 23, VL CDR2 composed of the amino acid sequence of SEQ ID NO: 30, and VL CDR3 composed of the amino acid sequence of SEQ ID NO: 35;
7) VH CDR1 composed of the amino acid sequence of SEQ ID NO: 2, VH CDR2 composed of the amino acid sequence of SEQ ID NO: 7, VH CDR3 composed of the amino acid sequence of SEQ ID NO: 14, VL CDR1 composed of the amino acid sequence of SEQ ID NO: 26, VL CDR2 composed of the amino acid sequence of SEQ ID NO: 31, and VL CDR3 composed of the amino acid sequence of SEQ ID NO: 38;
8) VH CDR1 composed of the amino acid sequence of SEQ ID NO: 2, VH CDR2 composed of the amino acid sequence of SEQ ID NO: 8, VH CDR3 composed of the amino acid sequence of SEQ ID NO: 14, VL CDR1 composed of the amino acid sequence of SEQ ID NO: 24, VL CDR2 composed of the amino acid sequence of SEQ ID NO: 30, and VL CDR3 composed of the amino acid sequence of SEQ ID NO: 38;
9) VH CDR1 composed of the amino acid sequence of SEQ ID NO: 2, VH CDR2 composed of the amino acid sequence of SEQ ID NO: 8, VH CDR3 composed of the amino acid sequence of SEQ ID NO: 18, VL CDR1 composed of the amino acid sequence of SEQ ID NO: 23, VL CDR2 composed of the amino acid sequence of SEQ ID NO: 29, and VL CDR3 composed of the amino acid sequence of SEQ ID NO: 40;
10) VH CDR1 composed of the amino acid sequence of SEQ ID NO: 2, VH CDR2 composed of the amino acid sequence of SEQ ID NO: 8, VH CDR3 composed of the amino acid sequence of SEQ ID NO: 17, VL CDR1 composed of the amino acid sequence of SEQ ID NO: 24, VL CDR2 composed of the amino acid sequence of SEQ ID NO: 27, and VL CDR3 composed of the amino acid sequence of SEQ ID NO: 38;
11) VH CDR1 composed of the amino acid sequence of SEQ ID NO: 2, VH CDR2 composed of the amino acid sequence of SEQ ID NO: 9, VH CDR3 composed of the amino acid sequence of SEQ ID NO: 18, VL CDR1 composed of the amino acid sequence of SEQ ID NO: 25, VL CDR2 composed of the amino acid sequence of SEQ ID NO: 27, and VL CDR3 composed of the amino acid sequence of SEQ ID NO: 38;
12) VH CDR1 composed of the amino acid sequence of SEQ ID NO: 2, VH CDR2 composed of the amino acid sequence of SEQ ID NO: 10, VH CDR3 composed of the amino acid sequence of SEQ ID NO: 19, VL CDR1 composed of the amino acid sequence of SEQ ID NO: 24, VL CDR2 composed of the amino acid sequence of SEQ ID NO: 29, and VL CDR3 composed of the amino acid sequence of SEQ ID NO: 35;
13) VH CDR1 composed of the amino acid sequence of SEQ ID NO: 2, VH CDR2 composed of the amino acid sequence of SEQ ID NO: 11, VH CDR3 composed of the amino acid sequence of SEQ ID NO: 18, VL CDR1 composed of the amino acid sequence of SEQ ID NO: 26, VL CDR2 composed of the amino acid sequence of SEQ ID NO: 29, and VL CDR3 composed of the amino acid sequence of SEQ ID NO: 36;
14) VH CDR1 composed of the amino acid sequence of SEQ ID NO: 2, VH CDR2 composed of the amino acid sequence of SEQ ID NO: 11, VH CDR3 composed of the amino acid sequence of SEQ ID NO: 14, VL CDR1 composed of the amino acid sequence of SEQ ID NO: 26, VL CDR2 composed of the amino acid sequence of SEQ ID NO: 32, and VL CDR3 composed of the amino acid sequence of SEQ ID NO: 34;
15) VH CDR1 composed of the amino acid sequence of SEQ ID NO: 2, VH CDR2 composed of the amino acid sequence of SEQ ID NO: 11, VH CDR3 composed of the amino acid sequence of SEQ ID NO: 18, VL CDR1 composed of the amino acid sequence of SEQ ID NO: 26, VL CDR2 composed of the amino acid sequence of SEQ ID NO: 27, and VL CDR3 composed of the amino acid sequence of SEQ ID NO: 41;
16) VH CDR1 composed of the amino acid sequence of SEQ ID NO: 2, VH CDR2 composed of the amino acid sequence of SEQ ID NO: 12, VH CDR3 composed of the amino acid sequence of SEQ ID NO: 20, VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 37;
17) VH CDR1 composed of the amino acid sequence of SEQ ID NO: 2, VH CDR2 composed of the amino acid sequence of SEQ ID NO: 12, VH CDR3 composed of the amino acid sequence of SEQ ID NO: 18, VL CDR1 composed of the amino acid sequence of SEQ ID NO: 25, VL CDR2 composed of the amino acid sequence of SEQ ID NO: 29, and VL CDR3 composed of the amino acid sequence of SEQ ID NO: 35;
18) VH CDR1 composed of the amino acid sequence of SEQ ID NO: 2, VH CDR2 composed of the amino acid sequence of SEQ ID NO: 12, VH CDR3 composed of the amino acid sequence of SEQ ID NO: 18, VL CDR1 composed of the amino acid sequence of SEQ ID NO: 25, VL CDR2 composed of the amino acid sequence of SEQ ID NO: 28, and VL CDR3 composed of the amino acid sequence of SEQ ID NO: 40;
19) VH CDR1 composed of the amino acid sequence of SEQ ID NO: 2, VH CDR2 composed of the amino acid sequence of SEQ ID NO: 12, VH CDR3 composed of the amino acid sequence of SEQ ID NO: 16, VL CDR1 composed of the amino acid sequence of SEQ ID NO: 25, VL CDR2 composed of the amino acid sequence of SEQ ID NO: 30, and VL CDR3 composed of the amino acid sequence of SEQ ID NO: 34;
20) VH CDR1 composed of the amino acid sequence of SEQ ID NO: 2, VH CDR2 composed of the amino acid sequence of SEQ ID NO: 13, VH CDR3 composed of the amino acid sequence of SEQ ID NO: 15, VL CDR1 composed of the amino acid sequence of SEQ ID NO: 23, VL CDR2 composed of the amino acid sequence of SEQ ID NO: 32, and VL CDR3 composed of the amino acid sequence of SEQ ID NO: 34;
21) VH CDR1 composed of the amino acid sequence of SEQ ID NO: 2, VH CDR2 composed of the amino acid sequence of SEQ ID NO: 13, VH CDR3 composed of the amino acid sequence of SEQ ID NO: 16, VL CDR1 composed of the amino acid sequence of SEQ ID NO: 22, VL CDR2 composed of the amino acid sequence of SEQ ID NO: 27, and VL CDR3 composed of the amino acid sequence of SEQ ID NO: 34;
22) VH CDR1 composed of the amino acid sequence of SEQ ID NO: 2, VH CDR2 composed of the amino acid sequence of SEQ ID NO: 13, VH CDR3 composed of the amino acid sequence of SEQ ID NO: 17, VL CDR1 composed of the amino acid sequence of SEQ ID NO: 22, VL CDR2 composed of the amino acid sequence of SEQ ID NO: 32, and VL CDR3 composed of the amino acid sequence of SEQ ID NO: 36;
23) VH CDR1 composed of the amino acid sequence of SEQ ID NO: 3, VH CDR2 composed of the amino acid sequence of SEQ ID NO: 7, VH CDR3 composed of the amino acid sequence of SEQ ID NO: 16, VL CDR1 composed of the amino acid sequence of SEQ ID NO: 25, VL CDR2 composed of the amino acid sequence of SEQ ID NO: 28, and VL CDR3 composed of the amino acid sequence of SEQ ID NO: 41;
24) VH CDR1 composed of the amino acid sequence of SEQ ID NO: 3, VH CDR2 composed of the amino acid sequence of SEQ ID NO: 7, VH CDR3 composed of the amino acid sequence of SEQ ID NO: 19, VL CDR1 composed of the amino acid sequence of SEQ ID NO: 23, VL CDR2 composed of the amino acid sequence of SEQ ID NO: 31, and VL CDR3 composed of the amino acid sequence of SEQ ID NO: 34;
25) VH CDR1 composed of the amino acid sequence of SEQ ID NO: 3, VH CDR2 composed of the amino acid sequence of SEQ ID NO: 8, VH CDR3 composed of the amino acid sequence of SEQ ID NO: 16, VL CDR1 composed of the amino acid sequence of SEQ ID NO: 22, VL CDR2 composed of the amino acid sequence of SEQ ID NO: 28, and VL CDR3 composed of the amino acid sequence of SEQ ID NO: 37;
26) VH CDR1 composed of the amino acid sequence of SEQ ID NO: 3, VH CDR2 composed of the amino acid sequence of SEQ ID NO: 8, VH CDR3 composed of the amino acid sequence of SEQ ID NO: 18, VL CDR1 composed of the amino acid sequence of SEQ ID NO: 22, VL CDR2 composed of the amino acid sequence of SEQ ID NO: 31, and VL CDR3 composed of the amino acid sequence of SEQ ID NO: 37;
27) VH CDR1 composed of the amino acid sequence of SEQ ID NO: 3, VH CDR2 composed of the amino acid sequence of SEQ ID NO: 8, VH CDR3 composed of the amino acid sequence of SEQ ID NO: 19, VL CDR1 composed of the amino acid sequence of SEQ ID NO: 25, VL CDR2 composed of the amino acid sequence of SEQ ID NO: 27, and VL CDR3 composed of the amino acid sequence of SEQ ID NO: 37;
28) VH CDR1 composed of the amino acid sequence of SEQ ID NO: 3, VH CDR2 composed of the amino acid sequence of SEQ ID NO: 9, VH CDR3 composed of the amino acid sequence of SEQ ID NO: 17, VL CDR1 composed of the amino acid sequence of SEQ ID NO: 23, VL CDR2 composed of the amino acid sequence of SEQ ID NO: 29, and VL CDR3 composed of the amino acid sequence of SEQ ID NO: 36;
29) VH CDR1 composed of the amino acid sequence of SEQ ID NO: 3, VH CDR2 composed of the amino acid sequence of SEQ ID NO: 9, VH CDR3 composed of the amino acid sequence of SEQ ID NO: 16, VL CDR1 composed of the amino acid sequence of SEQ ID NO: 21, VL CDR2 composed of the amino acid sequence of SEQ ID NO: 29, and VL CDR3 composed of the amino acid sequence of SEQ ID NO: 38;
30) VH CDR1 composed of the amino acid sequence of SEQ ID NO: 3, VH CDR2 composed of the amino acid sequence of SEQ ID NO: 10, VH CDR3 composed of the amino acid sequence of SEQ ID NO: 19, VL CDR1 composed of the amino acid sequence of SEQ ID NO: 22, VL CDR2 composed of the amino acid sequence of SEQ ID NO: 32, and VL CDR3 composed of the amino acid sequence of SEQ ID NO: 41;
31) VH CDR1 composed of the amino acid sequence of SEQ ID NO: 3, VH CDR2 composed of the amino acid sequence of SEQ ID NO: 12, VH CDR3 composed of the amino acid sequence of SEQ ID NO: 15, VL CDR1 composed of the amino acid sequence of SEQ ID NO: 24, VL CDR2 composed of the amino acid sequence of SEQ ID NO: 32, and VL CDR3 composed of the amino acid sequence of SEQ ID NO: 39;
32) VH CDR1 composed of the amino acid sequence of SEQ ID NO: 4, VH CDR2 composed of the amino acid sequence of SEQ ID NO: 7, VH CDR3 composed of the amino acid sequence of SEQ ID NO: 15, VL CDR1 composed of the amino acid sequence of SEQ ID NO: 25, VL CDR2 composed of the amino acid sequence of SEQ ID NO: 28, and VL CDR3 composed of the amino acid sequence of SEQ ID NO: 37;
33) VH CDR1 composed of the amino acid sequence of SEQ ID NO: 4, VH CDR2 composed of the amino acid sequence of SEQ ID NO: 7, VH CDR3 composed of the amino acid sequence of SEQ ID NO: 18, VL CDR1 composed of the amino acid sequence of SEQ ID NO: 23, VL CDR2 composed of the amino acid sequence of SEQ ID NO: 31, and VL CDR3 composed of the amino acid sequence of SEQ ID NO: 33;
34) VH CDR1 composed of the amino acid sequence of SEQ ID NO: 4, VH CDR2 composed of the amino acid sequence of SEQ ID NO: 10, VH CDR3 composed of the amino acid sequence of SEQ ID NO: 19, VL CDR1 composed of the amino acid sequence of SEQ ID NO: 21, VL CDR2 composed of the amino acid sequence of SEQ ID NO: 31, and VL CDR3 composed of the amino acid sequence of SEQ ID NO: 40;
35) VH CDR1 composed of the amino acid sequence of SEQ ID NO: 4, VH CDR2 composed of the amino acid sequence of SEQ ID NO: 10, VH CDR3 composed of the amino acid sequence of SEQ ID NO: 14, VL CDR1 composed of the amino acid sequence of SEQ ID NO: 24, VL CDR2 composed of the amino acid sequence of SEQ ID NO: 27, and VL CDR3 composed of the amino acid sequence of SEQ ID NO: 37;
36) VH CDR1 composed of the amino acid sequence of SEQ ID NO: 4, VH CDR2 composed of the amino acid sequence of SEQ ID NO: 12, VH CDR3 composed of the amino acid sequence of SEQ ID NO: 16, VL CDR1 composed of the amino acid sequence of SEQ ID NO: 23, VL CDR2 composed of the amino acid sequence of SEQ ID NO: 30, and VL CDR3 composed of the amino acid sequence of SEQ ID NO: 33;
37) VH CDR1 composed of the amino acid sequence of SEQ ID NO: 4, VH CDR2 composed of the amino acid sequence of SEQ ID NO: 12, VH CDR3 composed of the amino acid sequence of SEQ ID NO: 16, VL CDR1 composed of the amino acid sequence of SEQ ID NO: 24, VL CDR2 composed of the amino acid sequence of SEQ ID NO: 27, and VL CDR3 composed of the amino acid sequence of SEQ ID NO: 34;
38) VH CDR1 composed of the amino acid sequence of SEQ ID NO: 4, VH CDR2 composed of the amino acid sequence of SEQ ID NO: 13, VH CDR3 composed of the amino acid sequence of SEQ ID NO: 16, VL CDR1 composed of the amino acid sequence of SEQ ID NO: 22, VL CDR2 composed of the amino acid sequence of SEQ ID NO: 32, and VL CDR3 composed of the amino acid sequence of SEQ ID NO: 40;
39) VH CDR1 composed of the amino acid sequence of SEQ ID NO: 5, VH CDR2 composed of the amino acid sequence of SEQ ID NO: 7, VH CDR3 composed of the amino acid sequence of SEQ ID NO: 14, VL CDR1 composed of the amino acid sequence of SEQ ID NO: 21, VL CDR2 composed of the amino acid sequence of SEQ ID NO: 30, and VL CDR3 composed of the amino acid sequence of SEQ ID NO: 40;
40) VH CDR1 composed of the amino acid sequence of SEQ ID NO: 5, VH CDR2 composed of the amino acid sequence of SEQ ID NO: 7, VH CDR3 composed of the amino acid sequence of SEQ ID NO: 17, VL CDR1 composed of the amino acid sequence of SEQ ID NO: 23, VL CDR2 composed of the amino acid sequence of SEQ ID NO: 30, and VL CDR3 composed of the amino acid sequence of SEQ ID NO: 34;
41) VH CDR1 composed of the amino acid sequence of SEQ ID NO: 5, VH CDR2 composed of the amino acid sequence of SEQ ID NO: 7, VH CDR3 composed of the amino acid sequence of SEQ ID NO: 20, VL CDR1 composed of the amino acid sequence of SEQ ID NO: 21, VL CDR2 composed of the amino acid sequence of SEQ ID NO: 30, and VL CDR3 composed of the amino acid sequence of SEQ ID NO: 39;
42) VH CDR1 composed of the amino acid sequence of SEQ ID NO: 5, VH CDR2 composed of the amino acid sequence of SEQ ID NO: 8, VH CDR3 composed of the amino acid sequence of SEQ ID NO: 18, VL CDR1 composed of the amino acid sequence of SEQ ID NO: 21, VL CDR2 composed of the amino acid sequence of SEQ ID NO: 31, and VL CDR3 composed of the amino acid sequence of SEQ ID NO: 33;
43) VH CDR1 composed of the amino acid sequence of SEQ ID NO: 5, VH CDR2 composed of the amino acid sequence of SEQ ID NO: 10, VH CDR3 composed of the amino acid sequence of SEQ ID NO: 18, VL CDR1 composed of the amino acid sequence of SEQ ID NO: 21, VL CDR2 composed of the amino acid sequence of SEQ ID NO: 31, and VL CDR3 composed of the amino acid sequence of SEQ ID NO: 33;
44) VH CDR1 composed of the amino acid sequence of SEQ ID NO: 5, VH CDR2 composed of the amino acid sequence of SEQ ID NO: 10, VH CDR3 composed of the amino acid sequence of SEQ ID NO: 15, VL CDR1 composed of the amino acid sequence of SEQ ID NO: 21, VL CDR2 composed of the amino acid sequence of SEQ ID NO: 28, and VL CDR3 composed of the amino acid sequence of SEQ ID NO: 38;
45) VH CDR1 composed of the amino acid sequence of SEQ ID NO: 5, VH CDR2 composed of the amino acid sequence of SEQ ID NO: 10, VH CDR3 composed of the amino acid sequence of SEQ ID NO: 19, VL CDR1 composed of the amino acid sequence of SEQ ID NO: 26, VL CDR2 composed of the amino acid sequence of SEQ ID NO: 28, and VL CDR3 composed of the amino acid sequence of SEQ ID NO: 40;
46) VH CDR1 composed of the amino acid sequence of SEQ ID NO: 5, VH CDR2 composed of the amino acid sequence of SEQ ID NO: 11, VH CDR3 composed of the amino acid sequence of SEQ ID NO: 14, VL CDR1 composed of the amino acid sequence of SEQ ID NO: 21, VL CDR2 composed of the amino acid sequence of SEQ ID NO: 31, and VL CDR3 composed of the amino acid sequence of SEQ ID NO: 33;
47) VH CDR1 composed of the amino acid sequence of SEQ ID NO: 5, VH CDR2 composed of the amino acid sequence of SEQ ID NO: 11, VH CDR3 composed of the amino acid sequence of SEQ ID NO: 16, VL CDR1 composed of the amino acid sequence of SEQ ID NO: 23, VL CDR2 composed of the amino acid sequence of SEQ ID NO: 31, and VL CDR3 composed of the amino acid sequence of SEQ ID NO: 36;
48) VH CDR1 composed of the amino acid sequence of SEQ ID NO: 5, VH CDR2 composed of the amino acid sequence of SEQ ID NO: 11, VH CDR3 composed of the amino acid sequence of SEQ ID NO: 17, VL CDR1 composed of the amino acid sequence of SEQ ID NO: 23, VL CDR2 composed of the amino acid sequence of SEQ ID NO: 28, and VL CDR3 composed of the amino acid sequence of SEQ ID NO: 39;
49) VH CDR1 composed of the amino acid sequence of SEQ ID NO: 5, VH CDR2 composed of the amino acid sequence of SEQ ID NO: 11, VH CDR3 composed of the amino acid sequence of SEQ ID NO: 16, VL CDR1 composed of the amino acid sequence of SEQ ID NO: 25, VL CDR2 composed of the amino acid sequence of SEQ ID NO: 29, and VL CDR3 composed of the amino acid sequence of SEQ ID NO: 38;
50) VH CDR1 composed of the amino acid sequence of SEQ ID NO: 5, VH CDR2 composed of the amino acid sequence of SEQ ID NO: 12, VH CDR3 composed of the amino acid sequence of SEQ ID NO: 17, VL CDR1 composed of the amino acid sequence of SEQ ID NO: 21, VL CDR2 composed of the amino acid sequence of SEQ ID NO: 27, and VL CDR3 composed of the amino acid sequence of SEQ ID NO: 37;
51) VH CDR1 composed of the amino acid sequence of SEQ ID NO: 5, VH CDR2 composed of the amino acid sequence of SEQ ID NO: 12, VH CDR3 composed of the amino acid sequence of SEQ ID NO: 19, VL CDR1 composed of the amino acid sequence of SEQ ID NO: 26, VL CDR2 composed of the amino acid sequence of SEQ ID NO: 31, and VL CDR3 composed of the amino acid sequence of SEQ ID NO: 39;
52) VH CDR1 composed of the amino acid sequence of SEQ ID NO: 5, VH CDR2 composed of the amino acid sequence of SEQ ID NO: 12, VH CDR3 composed of the amino acid sequence of SEQ ID NO: 16, VL CDR1 composed of the amino acid sequence of SEQ ID NO: 23, VL CDR2 composed of the amino acid sequence of SEQ ID NO: 27, and VL CDR3 composed of the amino acid sequence of SEQ ID NO: 34;
53) VH CDR1 composed of the amino acid sequence of SEQ ID NO: 5, VH CDR2 composed of the amino acid sequence of SEQ ID NO: 12, VH CDR3 composed of the amino acid sequence of SEQ ID NO: 19, VL CDR1 composed of the amino acid sequence of SEQ ID NO: 24, VL CDR2 composed of the amino acid sequence of SEQ ID NO: 31, and VL CDR3 composed of the amino acid sequence of SEQ ID NO: 38;
54) VH CDR1 composed of the amino acid sequence of SEQ ID NO: 5, VH CDR2 composed of the amino acid sequence of SEQ ID NO: 13, VH CDR3 composed of the amino acid sequence of SEQ ID NO: 15, VL CDR1 composed of the amino acid sequence of SEQ ID NO: 26, VL CDR2 composed of the amino acid sequence of SEQ ID NO: 30, and VL CDR3 composed of the amino acid sequence of SEQ ID NO: 39;
55) VH CDR1 composed of the amino acid sequence of SEQ ID NO: 5, VH CDR2 composed of the amino acid sequence of SEQ ID NO: 13, VH CDR3 composed of the amino acid sequence of SEQ ID NO: 15, VL CDR1 composed of the amino acid sequence of SEQ ID NO: 26, VL CDR2 composed of the amino acid sequence of SEQ ID NO: 28, and VL CDR3 composed of the amino acid sequence of SEQ ID NO: 33;
56) VH CDR1 composed of the amino acid sequence of SEQ ID NO: 5, VH CDR2 composed of the amino acid sequence of SEQ ID NO: 13, VH CDR3 composed of the amino acid sequence of SEQ ID NO: 18, VL CDR1 composed of the amino acid sequence of SEQ ID NO: 21, VL CDR2 composed of the amino acid sequence of SEQ ID NO: 30, and VL CDR3 composed of the amino acid sequence of SEQ ID NO: 39;
57) VH CDR1 composed of the amino acid sequence of SEQ ID NO: 5, VH CDR2 composed of the amino acid sequence of SEQ ID NO: 13, VH CDR3 composed of the amino acid sequence of SEQ ID NO: 20, VL CDR1 composed of the amino acid sequence of SEQ ID NO: 25, VL CDR2 composed of the amino acid sequence of SEQ ID NO: 32, and VL CDR3 composed of the amino acid sequence of SEQ ID NO: 39;
58) VH CDR1 composed of the amino acid sequence of SEQ ID NO: 6, VH CDR2 composed of the amino acid sequence of SEQ ID NO: 7, VH CDR3 composed of the amino acid sequence of SEQ ID NO: 18, VL CDR1 composed of the amino acid sequence of SEQ ID NO: 25, VL CDR2 composed of the amino acid sequence of SEQ ID NO: 32, and VL CDR3 composed of the amino acid sequence of SEQ ID NO: 39;
59) VH CDR1 composed of the amino acid sequence of SEQ ID NO: 6, VH CDR2 composed of the amino acid sequence of SEQ ID NO: 10, VH CDR3 composed of the amino acid sequence of SEQ ID NO: 14, VL CDR1 composed of the amino acid sequence of SEQ ID NO: 25, VL CDR2 composed of the amino acid sequence of SEQ ID NO: 32, and VL CDR3 composed of the amino acid sequence of SEQ ID NO: 39;
60) VH CDR1 composed of the amino acid sequence of SEQ ID NO: 6, VH CDR2 composed of the amino acid sequence of SEQ ID NO: 11, VH CDR3 composed of the amino acid sequence of SEQ ID NO: 18, VL CDR1 composed of the amino acid sequence of SEQ ID NO: 22, VL CDR2 composed of the amino acid sequence of SEQ ID NO: 29, and VL CDR3 composed of the amino acid sequence of SEQ ID NO: 39;
61) VH CDR1 composed of the amino acid sequence of SEQ ID NO: 6, VH CDR2 composed of the amino acid sequence of SEQ ID NO: 11, VH CDR3 composed of the amino acid sequence of SEQ ID NO: 14, VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 39; or
62) VH CDR1 composed of the amino acid sequence of SEQ ID NO: 6, VH CDR2 composed of the amino acid sequence of SEQ ID NO: 13, VH CDR3 composed of the amino acid sequence of SEQ ID NO: 20, VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 38.

In some embodiments of the second monoclonal antibody or the antigen-binding antibody fragment thereof of the disclosure, the second monoclonal antibody or the antigen-binding antibody fragment thereof specifically recognizes an epitope composed of the amino acid sequence of SEQ ID No. 42, comprising:
(a) VH comprising VH CDR1 composed of the amino acid sequence of SEQ ID NO: 1, VH CDR2 composed of the amino acid sequence of SEQ ID NO: 12, and VH CDR3 composed of the amino acid sequence of SEQ ID NO: 17, and VL comprising VL CDR1 composed of the amino acid sequence of SEQ ID NO: 21, VL CDR2 composed of the amino acid sequence of SEQ ID NO: 32, and VL CDR3 composed of the amino acid sequence of SEQ ID NO: 33;
(b) VH comprising VH CDR1 composed of the amino acid sequence of SEQ ID NO: 2, VH CDR2 composed of the amino acid sequence of SEQ ID NO: 12, and VH CDR3 composed of the amino acid sequence of SEQ ID NO: 20, and VL comprising VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 37;
(c) VH comprising VH CDR1 composed of the amino acid sequence of SEQ ID NO: 3, VH CDR2 composed of the amino acid sequence of SEQ ID NO: 9, and VH CDR3 composed of the amino acid sequence of SEQ ID NO: 16, and VL comprising VL CDR1 composed of the amino acid sequence of SEQ ID NO: 21, VL CDR2 composed of the amino acid sequence of SEQ ID NO: 29, and VL CDR3 composed of the amino acid sequence of SEQ ID NO: 38;
(d) VH comprising VH CDR1 composed of the amino acid sequence of SEQ ID NO: 5, VH CDR2 composed of the amino acid sequence of SEQ ID NO: 13, and VH CDR3 composed of the amino acid sequence of SEQ ID NO: 18, and VL comprising VL CDR1 composed of the amino acid sequence of SEQ ID NO: 21, VL CDR2 composed of the amino acid sequence of SEQ ID NO: 30, and VL CDR3 composed of the amino acid sequence of SEQ ID NO: 39;
(e) VH comprising VH CDR1 composed of the amino acid sequence of SEQ ID NO: 4, VH CDR2 composed of the amino acid sequence of SEQ ID NO: 12, and VH CDR3 composed of the amino acid sequence of SEQ ID NO: 16, and VL comprising VL CDR1 composed of the amino acid sequence of SEQ ID NO: 23, VL CDR2 composed of the amino acid sequence of SEQ ID NO: 30, and VL CDR3 composed of the amino acid sequence of SEQ ID NO: 33; or
(f) VH comprising VH CDR1 composed of the amino acid sequence of SEQ ID NO: 6, VH CDR2 composed of the amino acid sequence of SEQ ID NO: 13, and VH CDR3 composed of the amino acid sequence of SEQ ID NO: 20, and VL comprising VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 38.

In some embodiments of the second monoclonal antibody or the antigen-binding antibody fragment thereof of the disclosure, the second monoclonal antibody or the antigen-binding antibody fragment thereof is an anti-soluble CD 14 subtype monoclonal antibody or an antigen-binding antibody fragment thereof secreted and produced by a hybridoma cell strain with the deposit number of CGMCC NO. 18536.

In some embodiments of the second monoclonal antibody or the antigen-binding antibody fragment thereof of the disclosure, the second monoclonal antibody or the antigen-binding antibody fragment thereof does not specifically bind to a soluble CD14 with a high molecular weight.

In some embodiments of the second monoclonal antibody or the antigen-binding antibody fragment thereof of the disclosure, the antigen-binding antibody fragment may be an antigen-binding antibody fragment selected from a group consisting of Fab, Fab', F(ab')2, a single-chain antibody (scFV), a dimerized V region (a dimeric antibody), a disulfide stabilized V region (dsFv), sc(Fv)2, a CDR-containing polypeptide, a heavy chain variable region-containing polypeptide, and a light chain variable region-containing polypeptide.

Detailed information about the second monoclonal antibody of the disclosure is described in the applicant's prior patent application PCT/CN 2019/119536, which is incorporated herein by reference in its entirety.

The kit according to the second aspect of the disclosure may also include other reagent components, including but not limited to a primary antibody, a secondary antibody, a labelled antibody, a labelled enzyme and other labelled substances; a chromogenic substrate, a fluorescent substrate, a chemiluminescent substrate, a specific binding substance such as biotin-streptavidin, an insoluble carrier, a blocking agent, a diluent, a washing solution, a standard substance, etc.

For example, a kit for use in a chemiluminescent enzyme immunoassay (CLEIA) may include an antibody coated on a solid phase, an enzyme-labelled antibody, a chemiluminescent substrate, a diluent, a washing solution, etc. Of course, in other embodiments, the antibody of the embodiments of the disclosure may also be used to prepare an enzyme immunoassay kit, an electrochemiluminescence immunoassay kit, an immunochromatography kit, etc. based on a double-antibody sandwich method or a competition method.

In some embodiments of the second aspect of the disclosure, the first monoclonal antibody or the antigen-binding antibody fragment thereof according to the disclosure is used as a capture antibody and the second monoclonal antibody or the antigen-binding antibody fragment thereof according to the disclosure is used as a detection antibody, or the second monoclonal antibody or the antigen-binding antibody fragment thereof according to the disclosure is used as a capture antibody and the first monoclonal antibody or the antigen-binding antibody fragment thereof according to the disclosure is used as a detection antibody, where the capture antibody is coated on a solid phase carrier and used to capture the soluble CD14 subtype in a sample to be tested.

In some embodiments of the second aspect of the disclosure, the solid phase carrier may be selected from a magnetic microsphere, a chip, a test paper, etc., preferably a magnetic microsphere, and more preferably a superparamagnetic bead.

In some embodiments of the second aspect of the disclosure, the signal label for the soluble CD14 subtype detection antibody may be, for example, a chemiluminescent label (such as an alkaline phosphatase, luminol, isoluminol, an acridinium ester, and a horseradish peroxidase), an electrochemiluminescent label (e.g., ruthenium terpyridine), a quantum dot (such as a gold quantum dot, a CdSe quantum dot, and a ZnCdSe quantum dot), a fluorescent microsphere, or a combination thereof.

The third aspect of the disclosure relates to a method for detecting a soluble CD14 subtype, including contacting the first monoclonal antibody or the antigen-binding antibody fragment thereof of the disclosure with a sample to be tested.

A method for immunoassay of a soluble CD14 subtype in a sample by using the first monoclonal antibody or the antigen-binding antibody fragment thereof of the disclosure may be, for example, an enzyme-linked immunosorbent assay (ELISA), a chemiluminescent enzyme immunoassay (CLEIA), a chemiluminescence immunoassay (CLIA), a fluorescent antibody test (FAT), a fluorescence enzyme immunoassay (FEIA), an electrochemiluminescence immunoassay (ECLIA), a radioimmunoassay (RIA), an immunochromatography method, an agglutination method, a competition method, etc., but not limited to these methods. The embodiments of the disclosure are particularly suitable for use in a chemiluminescent enzyme immunoassay.

ELISA (enzyme-linked immunosorbent assay) is one of the immunoassays using enzyme-labelled antibodies, including direct ELISA and indirect ELISA, for example, sandwich ELISA. The principle of the sandwich ELISA is as follows: antibodies with different antigen recognition sites are used, one of which is coated on a solid phase, and the other is used to sandwich an antigen to be tested, thereby forming an antibodyantigen-antibody complex.

The principle of the chemiluminescent enzyme immunoassay is as follows: an antigen in a sample is reacted with an antibody immobilized on a solid phase, and then reacted with an enzyme-labelled antibody; the mixture is washed, and then a chemiluminescent substrate is added thereto for enzymatic reaction, followed by determination of the luminescence intensity.

In some embodiments of the third aspect of the disclosure, the method includes:
mixing a sample to be tested with a solid phase support coated with a capture antibody, so that the capture antibody coated on the solid phase support binds to the soluble CD14 subtype in the sample to be tested;
washing the obtained mixture to remove unbound substances;
adding a labelled detection antibody to the washed mixture and mixing evenly, so that the detection antibody binds to the soluble CD14 subtype bound on the capture antibody to form a sandwich complex;
where the capture antibody or the detection antibody is the first monoclonal antibody or the antigen-binding antibody fragment thereof according to the disclosure;
washing the sandwich complex to remove unbound substances;
adding a detection substrate to the washed sandwich complex to detect the concentration of the soluble CD14 subtype in the sample. For example, a chemiluminescent substrate is added to the washed sandwich complex to detect the number of photons generated by the reaction, thereby obtaining a chemiluminescent signal value of the sample, which is directly proportional to the concentration of the soluble CD14 subtype.

Preferably, in some embodiments of the third aspect of the disclosure, a detection antibody may be the first monoclonal antibody or the antigen-binding antibody fragment thereof according to the disclosure. In these embodiments, a capture antibody may be the second monoclonal antibody or the antigen-binding antibody fragment thereof according to the disclosure.

Of course, in other alternative embodiments of the third aspect of the disclosure, a capture antibody may be the first monoclonal antibody or the antigen-binding antibody fragment thereof or the antigen-binding fragment thereof according to the disclosure. In this case, a detection antibody may be the second monoclonal antibody or the antigen-binding antibody fragment thereof according to the disclosure, or may be other anti-soluble CD14 subtype polyclonal antibodies.

In some embodiments of the third aspect of the disclosure, the sample to be tested may be blood, a blood component, such as whole blood, serum or plasma, preferably a plasma sample. However, the sample of the disclosure is not limited thereto, and may also be body fluids such as urine, tissue fluid, lymph fluid, joint fluid, milk, cerebrospinal fluid, pus, saliva, tears, mucus, nasal fluid, sputum, ascites, water, and semen.

The fourth aspect of the disclosure relates to use of the first monoclonal antibody or the antigen-binding antibody fragment thereof of the disclosure in the preparation of an analysis reagent for identifying whether a patient suffers from sepsis, where the first monoclonal antibody or the antigen-binding antibody fragment thereof of the disclosure is used to detect the concentration of a soluble CD14 subtype in a blood sample from the patient, and the increase in the concentration of the soluble CD14 subtype relative to a reference level is associated with an increase in possibility that the patient suffers from sepsis.

In some embodiments of the fourth aspect of the disclosure, the concentration of the soluble CD14 subtype is compared with a critical value to determine whether the concentration of the soluble CD14 subtype is higher than the critical value, thereby evaluating whether a patient suffers from sepsis. The increase in the concentration of the soluble CD14 subtype relative to a reference level is positively correlated with an increase in possibility that the patient suffers from sepsis.

In some embodiments of the fourth aspect of the disclosure, the concentration of the soluble CD14 subtype in the blood sample from the patient is detected within 72 hours after the patient is suspected of suffering from sepsis.

Some examples of the disclosure are described below, and these examples are not intended to limit the disclosure, but to better illustrate the disclosure.

### Example 1: Preparation of an anti-soluble CD14 subtype antibody

### Step 1.1 Expression of a soluble CD14 subtype immunogen

The soluble CD14 subtype immunogen is expressed by using a prokaryotic expression system and a eukaryotic expression system, respectively. The corresponding process is described below in detail.

### (1) Expression of a soluble CD14 subtype immunogen by using a prokaryotic expression system

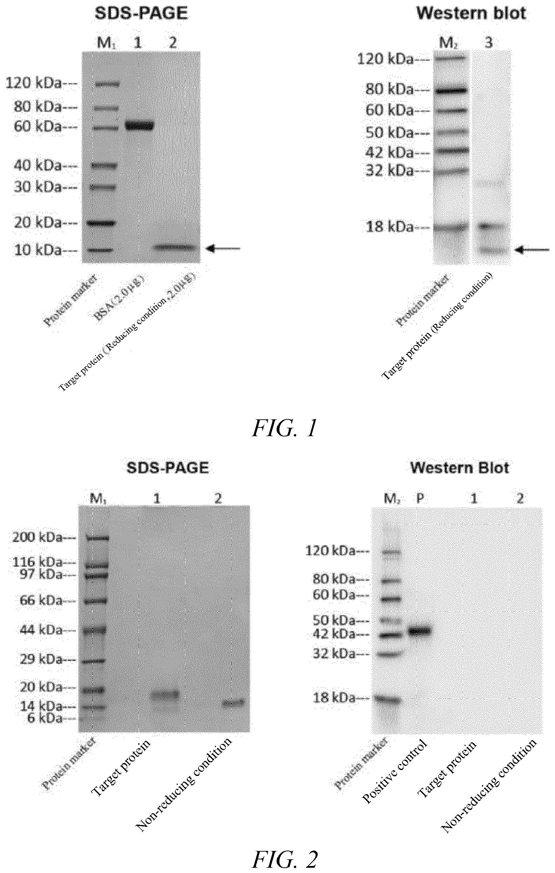

A target DNA sequence was synthesized, and then a pET30a expression vector was selected to construct a plasmid which was transformed into E. coli BL21star (DE3) to obtain a prokaryotic expression engineering strain. The recombinant plasmid was induced to be highly expressed in an E. coli expression system by an IPTG (isopropyl thiogalactoside) method. The target protein which mainly existed in an inclusion body, was purified and obtained by a Ni²⁺ NTA affinity column. The target protein had a sequence of Thr Thr Pro Glu Pro Cys Glu Leu Asp Asp Glu Asp Phe Arg Cys Val Cys Asn Phe Ser Glu Pro Gln Pro Asp Trp Ser Glu Ala Phe Gln Cys Val Ser Ala Val Glu Val Glu Ile His Ala Gly Gly Leu Asn Leu Glu Pro Phe Leu Lys Arg Val Asp Ala Asp Ala Asp Pro Arg Gln Tyr Ala (SEQ ID No. 43). As shown in FIG. 1, the concentration of the target protein was 5.42 mg/mL as determined by a Bradford method, and the purity and molecular weight thereof were determined by using SDS-PAGE and Western Blot.

### (2) Expression of a soluble CD14 subtype immunogen by using a eukaryotic expression system

A target DNA sequence was synthesized, and then a pcDNA3.4 expression vector was selected to construct a plasmid. An HEK293-6E expression system was used, and the culture conditions were as follows: the expression system was cultured in serumfree FreeStyle^{™} 293 Expression Medium (Thermo Fisher Scientific) matrix at 37°C and 5% CO₂. The constructed plasmid was transfected in the 293-6E expression system by using a transient transfection method. After 6 days of culture, a cell culture supernatant was collected. The supernatant was filtered and subjected to affinity purification. The purified protein was digested by using optimized experimental conditions, and a target protein was obtained after purification. As shown in FIG. 2, the concentration of the target protein was 1.55 mg/mL as determined by a Bradford method, and the purity and molecular weight thereof were determined by using SDS-PAGE and Western Blot.

### Step 1.2 Preparation of an anti-soluble CD14 subtype polyclonal antibody

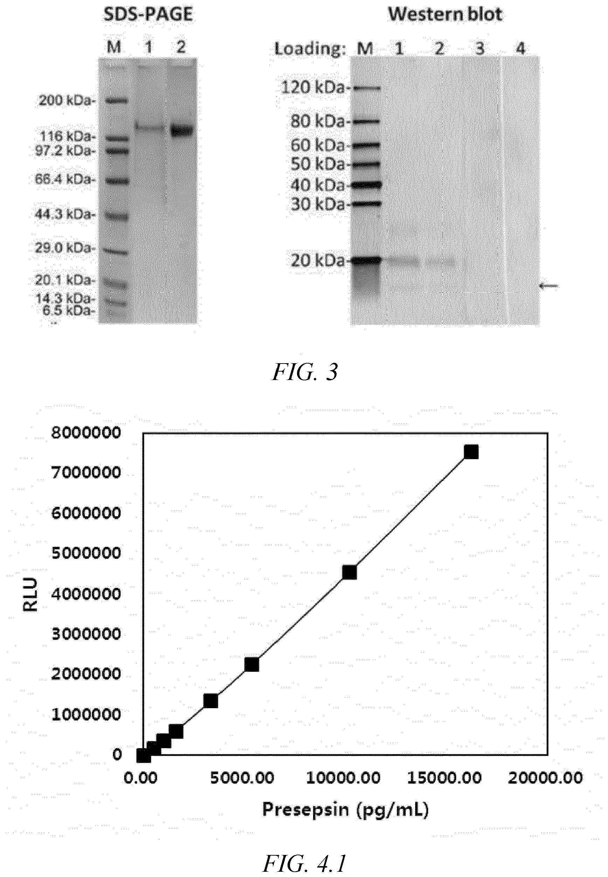

The proteins (i.e., antigens) expressed and purified by the prokaryotic system and the eukaryotic system obtained in Step 1.1 were mixed at a certain ratio, and the mixture was coupled with a carrier protein keyhole limpet hemocyanin (referred to as KLH for short), so that the resulting product was used as an immunogen to immunize New Zealand white rabbits according to the following steps: the immunogen was diluted with physiological saline, and then mixed with an adjuvant such as an incomplete Freund's adjuvant (Sigma-Aldrich) at 1 : 1. The antigen and the adjuvant were thoroughly mixed to form a stable emulsion, which was injected subcutaneously under the skin around the shoulders of New Zealand white rabbits and intramuscularly in the hind thighs thereof. Each region was immunized with about 1/4 of the immunogen, and the antigen amount of each immunization was about 100 µg to 500 µg, with a total of four immunizations and an interval of each immunization of 2 weeks. After the fourth immunization, the antiserum of New Zealand white rabbits was collected and subjected to antigen affinity purification to obtain a soluble CD14 subtype polyclonal antibody. The results of SDS-PAGE and Western Blot for the anti-soluble CD14 subtype polyclonal antibody are shown in FIG. 3. In the SDS-PAGE panel on the left side of FIG. 3, lane M is a Protein marker; the first lane is a polyclonal antibody; and the second lane is a rabbit IgG. In the Western Blot panel on the right side of FIG. 3, lane M is a Protein marker; the first lane is an immunogen (100 ng) + a purified antibody (1 µg/mL) + a goat anti-rabbit IgG [IRDye800cw] (0.125 µg/mL); the second lane is an immunogen (50 ng) + a purified antibody (1 µg/mL) + a goat anti-rabbit IgG [IRDye800cw] (0.125 µg/mL); the third lane is an immunogen (100 ng) + an unimmunized serum + a goat anti-rabbit IgG [IRDye800cw] (0.125 µg/mL); and the fourth lane is an immunogen (100 ng) + a goat anti-rabbit IgG [IRDye800cw] (0.125 µg/mL).

### Step 1.3 Preparation of an anti-soluble CD14 subtype monoclonal antibody as the second monoclonal antibody of the disclosure

The proteins (i.e., antigens) expressed and purified by the prokaryotic system and the eukaryotic system obtained in Step 1.1 were mixed at a certain ratio, and the mixture was coupled with a carrier protein keyhole limpet hemocyanin (referred to as KLH for short), so that the resulting product was used as an immunogen to immunize mice according to the following steps: at an initial immunization, the antigen (10 µg to 50 µg) was mixed with an adjuvant such as a complete Freund's adjuvant (Sigma-Aldrich) at 1 : 1, followed by subcutaneous multi-point injection. After 3 weeks, a second immunization was performed, whose dose was the same as that at the initial immunization, and an incomplete Freund's adjuvant (Sigma-Aldrich) was added for subcutaneous injection. After another 3 weeks, a third immunization was performed, whose dose was the same as that at the initial immunization, without the addition of an adjuvant. After 3 weeks, a booster immunization was performed at doses ranging from 100 µg to 500 µg. Three days after the booster immunization, the spleen was obtained for fusion. After the third immunization, the titre of antiserum in mice was evaluated by using an ELISA method, and the spleen cells of the animal with the best titre were selected to be fused with myeloma cells to obtain a parent clone cell strain, with the deposit number of CGMCC NO. 18536. In the scope of the disclosure, various well-known cells might be used as myeloma cells, for example, P3, NS-1, P3U1 and SP2/0 derived from mice, YB2/0 and Y3-Ag1 derived from rats, SKO-007 derived from human, and human-mouse hybrid myeloma cell SHM-D33. SP2/0 derived from mice was preferably used in the disclosure.

Then, the parent clone was screened positively and reversely by an ELISA method (an indirect ELISA method was used in both positive and reverse screening experiments).

For a positive screening experiment, the antigen was diluted with a coating buffer (a PBS buffer, pH = 7.4) to prepare into an antigen to be coated with a concentration of 1 µg/mL. 100 µL of the antigen to be coated was added to a 96-well plate and incubated at room temperature for 1 hour. The PBS buffer (pH = 7.4) was used as a washing solution to remove unreacted antigens. 100 µL of a blocking solution was added to the 96-well plate and incubated at room temperature for 1 hour to block the surface of unreacted solid phases. Excess blocking solution was removed again with the washing solution (the PBS buffer, pH = 7.4). Then, 100 µL of a cell supernatant to be screened was added and incubated at room temperature for 30 minutes. An antibody that did not bind to the solid-phase-coated antigen was removed by washing with the washing solution (the PBS buffer, pH = 7.4), and then 100 µL of a peroxidase-labelled goat anti-mouse antibody was added. The peroxidase-labelled goat anti-mouse antibody binded to a mouse antibody recognizing the solid phase antigen, and catalysed the luminescence of a substrate solution. The intensity of the luminescence was used to determine whether there was an antibody that might bind to the antigen in the cell supernatant.

During the antibody screening, the clone screened was confirmed to have no crossreaction with sCD14 by using sCD14 for reverse screening. For a reverse screening experiment, a His-tag protein and an sCD14 protein were diluted with a coating buffer (a PBS buffer, pH = 7.4), respectively to prepare into a substance to be coated with a concentration of 1 µg/mL. The subsequent experimental steps were the same as that of the positive screening experiment. The intensity of the final luminescence value was used to determine whether the antibody in the cell supernatant was reactive with a reverse screening substance.

Subsequent subcloning was performed after screening of the parent clone. Affinity sorting and epitope classification were performed on a subclone. The subclone supernatant was paired with the polyclonal antibody prepared in Step 1.2 to detect the antigen. The results of affinity sorting and epitope classification of the anti-soluble CD14 subtype monoclonal antibody, and of screening of the paired antibody are summarized in Tables 16 to 18. According to the results of affinity sorting and antibody pairing, an antibody with the clone number of 16D5B2 was screened for subsequent production and purification, thereby obtaining a mouse monoclonal antibody.

**Table 16**

| Affinity sorting | | |
|---|---|---|
| | Clone number | EC50 (ng/ml) |
| 1 | 16D5B2 | 1.163 |
| 2 | 16D5B12 | 1.194 |
| 3 | 16D5D6 | 1.284 |
| 4 | 3D5D9 | 1.404 |
| 5 | 3D5A2 | 1.512 |
| 6 | 3D5D8 | 1.680 |
| 7 | 11H1E11 | 1.998 |
| 8 | 11H1B9 | 2.356 |
| 9 | 11H1G11 | 2.661 |
| 10 | 17C7A6 | 5.493 |
| 11 | 17C7H7 | 9.137 |
| 12 | 20A10F7 | / |
| 13 | 20A10E6 | / |

**Table 17**

| Epitope classification | |
|---|---|
| Epitope 1 | Epitope 2 |
| 3D5A2 | 17C7A6 |
| 3D5D8 | 17C7H7 |
| 3D5D9 | |
| 16D5B2 | |
| 16D5B12 | |
| 16D5D6 | |
| 11H1B9 | |
| 11H1E11 | |
| 11H1G11 | |

**Table 18**

| Screening of paired antibody | | | | |
|---|---|---|---|---|
| | Clone number | Antigen concentration | Polyclonal antibody coating concentration | Corresponding value (RU) |
| 0 | Negative control (culture supernatant) | 100 nM | 5 µg/mL | -0.9 |
| 1 | 16D5B2 | | | 21.2 |
| 2 | 16D5B12 | | | 10.11 |
| 3 | 16D5D6 | | | 8.9 |
| 4 | 3D5D9 | | | 6.9 |
| 5 | 3D5A2 | | | 6.5 |
| 6 | 3D5D8 | | | 4.2 |
| 7 | 11H1E11 | | | -1.1 |
| 8 | 11H1B9 | | | 7.2 |
| 9 | 11H1G11 | | | -1.3 |
| 10 | 17C7A6 | | | 0.9 |
| 11 | 17C7H7 | | | 1.4 |

### Step 1.4: Determination of amino acid sequences of the anti-soluble CD14 subtype monoclonal antibody as the second monoclonal antibody of the disclosure

The variable regions of the anti-soluble CD14 subtype monoclonal antibody obtained from screening in Step 1.3 as the second monoclonal antibody of the disclosure were sequenced. First, the total RNA of hybridoma cells was extracted according to the instruction manual of TRIzol^{®} kit, and then the total RNA was reverse transcribed into cDNA using an epitope-specific antisense primer or universal primer according to the instruction manual of PrimeScript^{™} 1st Strand cDNA Synthesis Kit. The corresponding amino acid sequences of the anti-soluble CD14 subtype monoclonal antibody might be obtained by the reverse transcription-synthesized cDNA sequence. The anti-soluble CD14 subtype monoclonal antibody included, for example, VH comprising VH CDR1 composed of the amino acid sequence of SEQ ID NO: 3, VH CDR2 composed of the amino acid sequence of SEQ ID NO: 9 and VH CDR3 composed of the amino acid sequence of SEQ ID NO: 16, and VL comprising VL CDR1 composed of the amino acid sequence of SEQ ID NO: 21, VL CDR2 composed of the amino acid sequence of SEQ ID NO: 29 and VL CDR3 composed of the amino acid sequence of SEQ ID NO: 38, where the anti-soluble CD14 subtype monoclonal antibody specifically recognized an epitope composed of the amino acid sequence of SEQ ID No. 42.

### Step 1.5 Preparation of an anti-soluble CD14 subtype monoclonal antibody as the first monoclonal antibody of the disclosure

A parent clone cell strain with the deposit number of CGMCC NO. 19395 was obtained by the step similar to Step 1.3. Then, a parent clone supernatant containing a 16D5B2 paired antibody was screened using Biacore X100 (GE Healthcare). The specific experimental method included:
(1) Coupling of a 16D5B2 antibody: the 16D5B2 antibody was diluted to 1 µg/ml-20 µg/ml with an Acetate 4.0 buffer (GE Healthcare, BR-1003-49). With the "Immobilization" application program in Biacore X100, and following the prompts on the instrument, a solution in an amine coupling kit (GE Healthcare, BR-1000-50), the diluted antibody solution, and a NaOH solution were put into a sample disk, with the target coupling amount of 10000 RU-3000 RU.
(2) Screening of a supernatant containing a paired antibody: the Presepsin antigen obtained in Step 1.1 was diluted to 200 nM with 1 × HBS-EP + Buffer (GE Healthcare, BR-1006-69). After the 200 nM of the Presepsin antigen binding to a CM5-16D5B2 chip, the parent clone supernatant diluted IX with the HBS-EP Buffer was loaded, and the response value (RU) of each parent clone supernatant was observed.

The detection results were as shown in Table 19. The parent clone supernatants with the numbers of 3G8, 27F7 and 1B11 were screened out due to a higher response value (RU) and thus a greater possibility for pairing.

**Table 19**

| Number of parent clone supernatant | Response value (RU) of specific binding of parent clone supernatant to chip | Number of parent clone supernatant | Response value (RU) of specific binding of parent clone supernatant to chip |
|---|---|---|---|
| 1A10 | -0.9 | 10E2 | -4.4 |
| 1B11 | 14.1 | 10H4 | 2 |
| 1E4 | 0 | 15A8 | 0.1 |
| 1F1 | -0.2 | 8E3 | 0.8 |
| 3E2 | 1.1 | 11H1 | -0.3 |
| 1H3 | -0.9 | 11H3 | 0.2 |
| 2A11 | -1.7 | 11H11 | 2.6 |
| 3A2 | -2.4 | 15C2 | 3 |
| 3B7 | -0.8 | 16D5 | 6.8 |
| 3B3 | -1.4 | 17C7 | 0.9 |
| 3D5 | -0.5 | 30C2 | 1.2 |
| 3D9 | 0.5 | 26F12 | 2.2 |
| 3E12 | -3.8 | 28D7 | 2.6 |
| 8A2 | -0.5 | 20A10 | 0.8 |
| 3G8 | 34.7 | 26G7 | 0.5 |
| 4H6 | -1.1 | 27A7 | 2 |
| 5G7 | -1.9 | 17H10 | -1.5 |
| 3G10 | -0.3 | 27F17 | 24.2 |
| 8D6 | -0.7 | 17F8 | 1.1 |
| 6H7 | 0.2 | 20H11 | 0.3 |
| Anti-Presepsin polyclonal antibody | 29.1 | Negative control | 0.3 |

After the parent clones (with the numbers of 3G8, 27F7 and 1B11) were screened out, the subsequent subcloning was carried out and stable strains were established, thereby obtaining a total of 6 monoclonal antibodies, with the clone numbers of 3G8-1, 3G8-2, 27F7-1, 27F7-2, 1B11-1 and 1B11-2, respectively.

The monoclonal antibody with the clone number of 16D5B2 obtained in Step 1.3 (namely, the second monoclonal antibody of the disclosure) was used as the capture antibody and coated on the surface of a superparamagnetic bead (Merck, Estapor^{®} superparamagnetic bead). The surface of the superparamagnetic bead was modified with a carboxyl group, and the carboxyl group on the surface of the superparamagnetic bead was coupled with an amino group of the monoclonal antibody with the clone number of 16D5B2 under the catalysis of EDC/NHS, thereby preparing a coated magnetic bead. The coated magnetic bead was diluted in 50 mM of MES (2-morpholinoethanesulfonic acid) buffer (0.5 M NaCl, 0.5% BSA, 0.05% Tween 20, pH = 6.0) to a concentration of 1.0 mg/mL.

The 3G8-1, 3G8-2, 27F7-1, 27F7-2, 1B11-1, 1B11-2 and polyclonal antibody were respectively used as detection antibodies which were labelled with an alkaline phosphatase (Roche Life Science). The enzyme label conjugate was diluted in 50 mM of MES buffer (0.5 M NaCl, 0.5% BSA, 0.05% Tween 20, pH = 6.0) to a concentration of 1 µg/mL. The signal-to-noise ratios of the reagents were compared. It can be seen from Table 20 below that the signal-to-noise ratio is the highest when the clone number of 3G8-2 is selected. Therefore, an antibody with the clone number of 3G8-2 was screened for subsequent production and purification, thereby obtaining a mouse monoclonal antibody (namely, the first monoclonal antibody of the disclosure).

**Table 20**

| Signal-to-noise ratio of different combinations of antibodies | | | | | | | |
|---|---|---|---|---|---|---|---|
| Magnetic bead antibody | 16D5B2 | | | | | | |
| Enzyme-labelled antibody | 3G8-1 | 3G8-2 | 27F7-1 | 27F7-2 | 1B11-1 | 1B11-2 | Anti -Presepsin polyclonal antibody |
| Sample 1 | 1.37 | 1.79 | 1.16 | 1.13 | 1.05 | 1.05 | 1.42 |
| Sample 2 | 3.31 | 4.30 | 3.00 | 2.32 | 1.17 | 1.29 | 3.30 |
| Sample 3 | 7.09 | 9.00 | 6.43 | 5.59 | 2.32 | 2.21 | 7.80 |
| Sample 4 | 13.36 | 17.15 | 12.00 | 10.61 | 5.20 | 5.71 | 13.63 |
| Sample 5 | 68.12 | 86.77 | 60.57 | 48.81 | 19.79 | 17.26 | 69.91 |

### Step 1.6: Determination of amino acid sequences of the anti-soluble CD14 subtype monoclonal antibody as the first monoclonal antibody of the disclosure

The variable regions of the anti-soluble CD14 subtype monoclonal antibody obtained from screening in Step 1.5 as the first monoclonal antibody of the disclosure were sequenced. First, the total RNA of hybridoma cells was extracted according to the instruction manual of TRIzol^{®} kit, and then the total RNA was reverse transcribed into cDNA using an epitope-specific antisense primer or universal primer according to the instruction manual of PrimeScript^{™} 1st Strand cDNA Synthesis Kit. The corresponding amino acid sequences of the anti-soluble CD14 subtype monoclonal antibody might be obtained by the reverse transcription-synthesized cDNA sequence. The anti-soluble CD14 subtype monoclonal antibody included, for example, VH comprising VH CDR1 composed of the amino acid sequence of SEQ ID NO: 46, VH CDR2 composed of the amino acid sequence of SEQ ID NO: 52 and VH CDR3 composed of the amino acid sequence of SEQ ID NO: 61, and VL comprising VL CDR1 composed of the amino acid sequence of SEQ ID NO: 68, VL CDR2 composed of the amino acid sequence of SEQ ID NO: 71 and VL CDR3 composed of the amino acid sequence of SEQ ID NO: 83, where the anti-soluble CD14 subtype monoclonal antibody specifically recognized an epitope composed of the amino acid sequence of SEQ ID No. 84.

### Example 2: Determination of a specific epitope of an anti-soluble CD14 subtype monoclonal antibody

In the disclosure, a method for determining an epitope is not particularly defined, for example, it can be carried out in the following manner: firstly, a polypeptide was synthesized by successively moving 3 amino acids from the N-terminus to the C-terminus of the target protein sequence, and each peptide chain consisted of 15 amino acids; then, the polypeptide was labelled with biotin and coated on a 96-well plate containing streptavidin by biotin-streptavidin binding; next, an antibody to be detected and a peroxidase-labelled goat anti-mouse IgG were added to the 96-well plate; the amino acid sequences recognized by the antibody to be detected were determined by the signal value of the final reaction.

### Example 3: Preparation of a kit for detecting a soluble CD14 subtype

Example 3.1: Preparation of a kit for detecting a soluble CD14 subtype: the kit included the second monoclonal antibody of the disclosure (16D5B2) as a capture antibody and the anti-soluble CD14 subtype polyclonal antibody prepared in Step 1.2 as a detection antibody (also referred to herein as kit 1 for detecting a soluble CD14 subtype for short).

The monoclonal antibody with the clone number of 16D5B2 screened in Example 1 was used as a capture antibody and coated on the surface of a superparamagnetic bead (Merck, Estapor^{®} superparamagnetic bead). The surface of the superparamagnetic bead was modified with a carboxyl group, and the carboxyl group on the surface of the superparamagnetic bead was coupled with an amino group of the monoclonal antibody with the clone number of 16D5B2 under the catalysis of EDC/NHS, thereby preparing a coated magnetic bead. The coated magnetic bead was diluted in 50 mM of MES (2-morpholinoethanesulfonic acid) buffer (0.5 M NaCl, 0.5% BSA, 0.05% Tween 20, pH = 6.0) to a concentration of 1.0 mg/mL. The anti-soluble CD14 subtype polyclonal antibody prepared in Step 1.2 of Example 1 was used as a detection antibody which was labelled with an alkaline phosphatase (Roche Life Science). The enzyme label conjugate was diluted in 50 mM of MES buffer (0.5 M NaCl, 0.5% BSA, 0.05% Tween 20, pH = 6.0) to a concentration of 1 µg/mL.

In this example, the detection reaction was based on the sandwich format: a sample to be tested (40 µL), the capture antibody (50 µL) and the detection antibody (50 µL) were added to a reaction tube, and the mixture was incubated, where the sample to be tested was plasma. The antigen in the sample to be tested, that is, the soluble CD14 subtype, binded to the capture antibody and the detection antibody to form a sandwich complex. After the reaction was incubated in the reaction tube, magnetic beads were attracted by the magnetic field, thereby washing away unbound substances.

A chemiluminescent substrate (sodium 3-((1R,3S,5r,7r)- 4'-methoxyspiro[adamantane-2,3'-[1,2] dioxetan]- 4'-yl)phenyl phosphate, AMPPD) was added to the reaction tube, and decomposed by an alkaline phosphatase, and a phosphate group was removed to produce an unstable intermediate. The intermediate generated a methyl m-oxybenzoate anion by intra-molecular electron transfer, and the chemiluminescence was generated as the methyl m-oxybenzoate anion in the excited state returning to the ground state. Next, the number of photons produced by the intermediate reaction was measured by a photomultiplier tube, and the number of the photons produced was directly proportional to the concentration of the soluble CD14 subtypes in the sample. Finally, a series of samples containing soluble CD14 subtypes at different concentrations were tested to obtain the corresponding luminescence values thereof, and then a standard curve was established, as shown in FIG. 4.1.

**Example 3.2:** Preparation of a kit for detecting a soluble CD14 subtype: the kit included the first monoclonal antibody of the disclosure (3G8-2) and the second monoclonal antibody of the disclosure (16D5B2) (also referred to herein as kit 2 for detecting a soluble CD14 subtype for short).

In the existing kits for detecting Presepsin, a monoclonal antibody was generally used as a capture antibody, and a rabbit polyclonal antibody was used as a detection antibody. For example, in the kit in Example 3.1 of the disclosure, the first monoclonal antibody of the disclosure (3G8-2) might be used as a capture antibody and a rabbit polyclonal antibody might be used as a detection antibody. The rabbit polyclonal antibody was obtained by collecting the antiserum of New Zealand rabbits immunized with a Presepsin antigen and subjecting the antiserum to antigen affinity purification. However, batch-to-batch difference of rabbit polyclonal antibodies was difficult to control due to the large difference in immune responses among rabbits. In addition, since rabbits were small animals, the production of the antiserum thereof was low, which was not conducive to the continuous and stable production of reagents.

Therefore, the first monoclonal antibody of the disclosure (3G8-2) and the second monoclonal antibody of the disclosure (16D5B2) were preferably included in the kit 2 for detecting a soluble CD14 subtype of the disclosure. The monoclonal antibody with the clone number of 16D5B2 screened in Step 1.3 of Example 1 was used as a capture antibody and coated on the surface of a superparamagnetic bead (Merck, Estapor^{®} superparamagnetic bead). The surface of the superparamagnetic bead was modified with a carboxyl group, and the carboxyl group on the surface of the superparamagnetic bead was coupled with an amino group of the monoclonal antibody with the clone number of 16D5B2 under the catalysis of EDC/NHS, thereby preparing a coated magnetic bead. The coated magnetic bead was diluted in 50 mM of MES (2-morpholinoethanesulfonic acid) buffer (0.5 M NaCl, 0.5% BSA, 0.05% Tween 20, pH = 6.0) to a concentration of 1.0 mg/mL. The monoclonal antibody with the clone number of 3G8-2 screened in Step 1.5 of Example 1 was used as a detection antibody which was labelled with an alkaline phosphatase (Roche Life Science). The enzyme label conjugate was diluted in 50 mM of MES buffer (0.5 M NaCl, 0.5% BSA, 0.05% Tween 20, pH = 6.0) to a concentration of 1 µg/mL.

In this example, the detection reaction was based on the sandwich format: a sample to be tested (40 µL), the capture antibody (50 µL) and the detection antibody (50 µL) were added to a reaction tube, and the mixture was incubated, where the sample to be tested was plasma. The antigen in the sample to be tested, that is, the soluble CD14 subtype, binded to the capture antibody and the detection antibody to form a sandwich complex. After the reaction was incubated in the reaction tube, magnetic beads were attracted by the magnetic field, thereby washing away unbound substances.

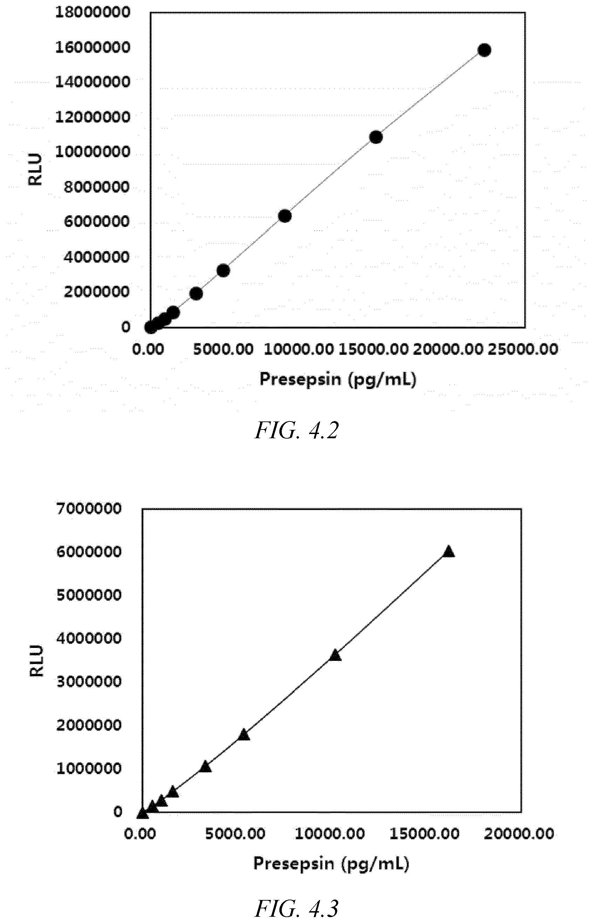

A chemiluminescent substrate (sodium 3-((1R,3S,5r,7r)- 4'-methoxyspiro[adamantane-2,3'-[1,2] dioxetan]- 4'-yl)phenyl phosphate, AMPPD) was added to the reaction tube, and decomposed by an alkaline phosphatase, and a phosphate group was removed to produce an unstable intermediate. The intermediate generated a methyl m-oxybenzoate anion by intra-molecular electron transfer, and the chemiluminescence was generated as the methyl m-oxybenzoate anion in the excited state returning to the ground state. Next, the number of photons produced by the intermediate reaction was measured by a photomultiplier tube, and the number of the photons produced was directly proportional to the concentration of the soluble CD14 subtypes in the sample. Finally, a series of samples containing soluble CD14 subtypes at different concentrations were tested to obtain the corresponding luminescence values thereof, and then a standard curve was established, as shown in FIG. 4.2.

According to the disclosure, since the monoclonal antibody with the clone number of 16D5B2 was paired with the monoclonal antibody with the clone number of 3G8-2, in the kit 2 for detecting a soluble CD14 subtype of the disclosure, the monoclonal antibody with the clone number of 3G8-2 might also be used as a capture antibody, and the monoclonal antibody with the clone number of 16D5B2 was used as a detection antibody.

**Example 3.3:** Preparation of a kit for detecting a soluble CD14 subtype: the kit included the first monoclonal antibody of the disclosure (3G8-2) and the anti-soluble CD14 subtype polyclonal antibody prepared in Step 1.2 as a detection antibody (also referred to herein as kit 3 for detecting a soluble CD14 subtype for short).

The monoclonal antibody with the clone number of 3G8-2 screened in Example 1 was used as a capture antibody and coated on the surface of a superparamagnetic bead (Merck, Estapor^{®} superparamagnetic bead). The surface of the superparamagnetic bead was modified with a carboxyl group, and the carboxyl group on the surface of the superparamagnetic bead was coupled with an amino group of the monoclonal antibody with the clone number of 3G8-2 under the catalysis of EDC/NHS, thereby preparing a coated magnetic bead. The coated magnetic bead was diluted in 50 mM of MES (2-morpholinoethanesulfonic acid) buffer (0.5 M NaCl, 0.5% BSA, 0.05% Tween 20, pH = 6.0) to a concentration of 1.0 mg/mL. The anti-soluble CD14 subtype polyclonal antibody prepared in Step 1.2 of Example 1 was used as a detection antibody which was labelled with an alkaline phosphatase (Roche Life Science). The enzyme label conjugate was diluted in 50 mM of MES buffer (0.5 M NaCl, 0.5% BSA, 0.05% Tween 20, pH = 6.0) to a concentration of 1 µg/mL.

In this example, the detection reaction was based on the sandwich format: a sample to be tested (40 µL), the capture antibody (50 µL) and the detection antibody (50 µL) were added to a reaction tube, and the mixture was incubated, where the sample to be tested was plasma. The antigen in the sample to be tested, that is, the soluble CD14 subtype, binded to the capture antibody and the detection antibody to form a sandwich complex. After the reaction was incubated in the reaction tube, magnetic beads were attracted by the magnetic field, thereby washing away unbound substances.

A chemiluminescent substrate (sodium 3-((1R,3S,5r,7r)- 4'-methoxyspiro[adamantane-2,3'-[1,2] dioxetan]- 4'-yl)phenyl phosphate, AMPPD) was added to the reaction tube, and decomposed by an alkaline phosphatase, and a phosphate group was removed to produce an unstable intermediate. The intermediate generated a methyl m-oxybenzoate anion by intra-molecular electron transfer, and the chemiluminescence was generated as the methyl m-oxybenzoate anion in the excited state returning to the ground state. Next, the number of photons produced by the intermediate reaction was measured by a photomultiplier tube, and the number of the photons produced was directly proportional to the concentration of the soluble CD14 subtypes in the sample. Finally, a series of samples containing soluble CD14 subtypes at different concentrations were tested to obtain the corresponding luminescence values thereof, and then a standard curve was established, as shown in FIG. 4.3.

### Example 3.4: Test of comparison of consistency among the above-mentioned kit 1, kit 2 and kit 3

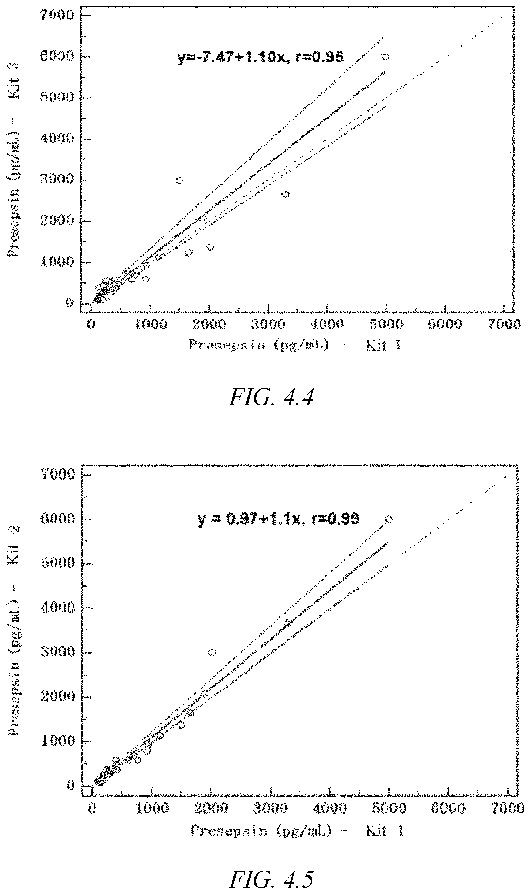

The kit 1, kit 2 and kit 3 prepared in Examples 3.1 to 3.3 were used to detect 50 samples, respectively. The detection results were as shown in FIG. 4.4 and FIG. 4.5: specifically, the correlation coefficient r of kit 1 and kit 2 was 0.99; and the correlation coefficient r of kit 1 and kit 3 was 0.95. In the methodological comparison, the correlation coefficient r reflects the consistency of the measurement values obtained by two methods, and if r > 0.90, the consistency between the two methods is considered to be good. The higher the correlation coefficient r, the better the consistency. Therefore, the consistency of the measurement values of the three kits are considered to be good, but the consistency between kit 2 and kit 1 is higher than that between kit 3 and kit 1.

### Example 4: Use of an anti-soluble CD14 subtype monoclonal antibody or an antigen-binding antibody fragment thereof in the preparation of an analysis reagent for identifying whether a patient suffers from sepsis

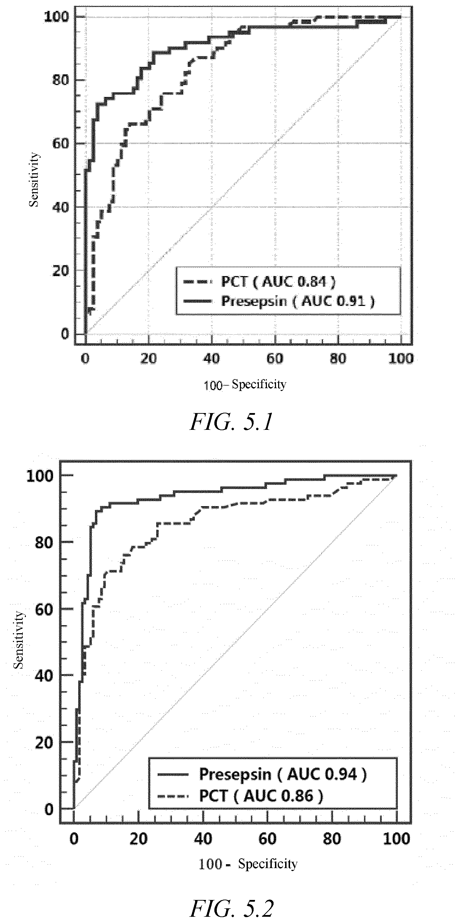

Samples from 141 suspected sepsis patients were detected for the soluble CD14 subtype using the kit prepared in Example 3.1. Moreover, the PCT concentration levels of the patients were detected, and the diagnostic power of the soluble CD14 subtype and PCT for sepsis was compared. As shown in FIG. 5.1, the AUC areas of the ROC curve for the soluble CD14 subtype and PCT were 0.91 and 0.84, respectively.

Plasma samples from 200 suspected sepsis patients within 72 hours after admission to hospital were detected for the soluble CD14 subtype using the kit for detecting a soluble CD14 subtype prepared in Example 3.2. Moreover, the PCT concentration levels of the patients were detected, and the diagnostic power of the soluble CD14 subtype and PCT for sepsis was compared. As shown in FIG. 5.2, the AUC areas of the ROC curve for the soluble CD14 subtype and PCT were 0.94 and 0.86, respectively. It was concluded therefrom that the soluble CD14 subtype had a higher diagnostic power.

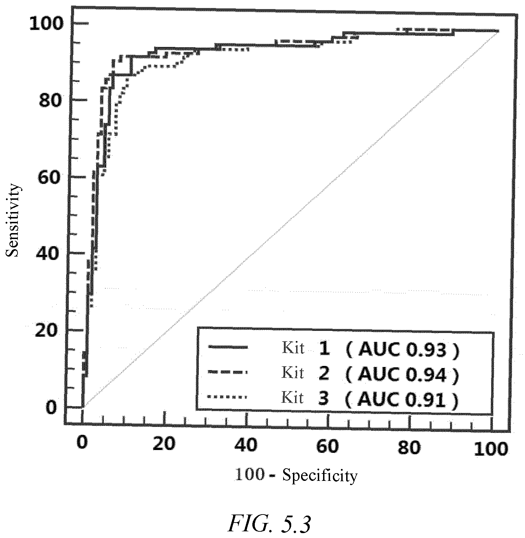

The diagnostic powers of the kit 1, kit 2, and kit 3 prepared in Examples 3.1 to 3.3 were compared. Plasma samples from 200 suspected sepsis patients within 72 hours after admission to hospital were detected for the soluble CD14 subtype using kit 1, kit 2 and kit 3, respectively. The diagnostic powers of kit 1, kit 2 and kit 3 were as shown in FIG 5.3, where the AUC areas of the ROC curve of kit 1, kit 2 and kit 3 were 0.93, 0.94 and 0.91, respectively.

The kit for detecting a soluble CD14 subtype prepared in Example 3.2 was used to detect 200 samples, including 60 healthy people, 52 people with local infection, 51 patients with sepsis, and 37 people with septic shock. The diagnostic criteria are based on Sepsis 3.0 (Singer M, Deutschman CS, Seymour CW, et al., The Third international consensus definitions for sepsis and septic shock (Sepsis-3) [J]), and the detection results of different groups (see Table 21) show that with the development of infection, the concentration value of Presepsin is increasing.

**Table 21**

| Group | Number of samples | Presepsin (pg/mL) | | |
|---|---|---|---|---|
| | | 2.5th percentile | Median | 97.5th percentile |
| Healthy people | 60 | 87.21 | 155.67 | 367.20 |
| People with local infection | 52 | 301.35 | 443.17 | 622.73 |
| Patients with sepsis | 51 | 767.12 | 915.23 | 1354.22 |
| People with septic shock | 37 | 932.24 | 1500.47 | 5931.81 |

From the various embodiments described above, it may be concluded that the anti-soluble CD14 subtype monoclonal antibody or the antigen-binding antibody fragment thereof of the embodiments of the disclosure or the kit of the embodiments of the disclosure has high specificity and sensitivity and is suitable for detecting the soluble CD14 subtype in a sample to be tested, thereby improving the detection quality and precision for the soluble CD14 subtype.

The disclosure is not limited to this by the description based on the embodiments. In particular, the disclosure includes each new feature and any combination of the features, especially any combination of features contained in the claims, even if the feature or the combination itself is not specified in detail in the claims or the embodiments.

## Claims

1. An anti-soluble CD14 subtype monoclonal antibody or an antigen-binding antibody fragment thereof, which specifically recognizes an epitope composed of an amino acid sequence of SEQ ID No. 84, wherein
the anti-soluble CD14 subtype monoclonal antibody or the antigen-binding antibody fragment thereof comprises:
(i) heavy chain variable region (VH) complementarity determining regions (CDRs):
VH CDR1: X₁X₂X₃MX₄;
VH CDR2: RIX₅X₆DPX₇; and
VH CDR3: X₈X₉X₁₀RX₁₁;
and
(ii) light chain variable region (VL) complementarity determining regions (CDRs):
VL CDR1: KX₁₂X₁₃X₁₄LA;
VL CDR2: WX₁₅X₁₆; and
VL CDR3: KQX₁₇X₁₈;
wherein X₁ to X₁₈ are one or more of amino acid sequences listed below as options:
X₁ = any one of amino acids;
X₂ = T or V; X₃ = A, Y or L; X₄ = Q or G;
X₅ = TYANG, DPMGG, SPASS, DPANG, or SPANS;
X₆ = GTKA, TTKS, NTKY, TTKS or TTKA;
X₇ = KFQA, TKFG, SQFA, MKFG or KFNS;
X₈ = F or G; X₉ = GG, GQ or CG; X₁₀ = YN, YV or KF; X₁₁ = FDY, FYY, FDC or TDC;
X₁₂ = SSAS, STAT, SSQS or STSS;
X₁₃ = LLKS, LLAS, LLSS or KASS;
X₁₄ = NRNIHL, RTRKHY, NRKHYL or YTNKAL;
X₁₅ = ATS, AST, QSS or ASK;
X₁₆ = RAS, LDS, LEA or RES;
X₁₇ = GYN, YNA, YLN or SYN;
X₁₈ = LWT, LNT, GNA or TWI.

2. The anti-soluble CD14 subtype monoclonal antibody or the antigen-binding antibody fragment thereof of claim 1, wherein
the anti-soluble CD14 subtype monoclonal antibody or the antigen-binding antibody fragment thereof comprises VH CDR1, VH CDR2 and VH CDR3, as well as VL CDR1, VL CDR2 and VL CDR3,
wherein VH CDR1, VH CDR2 and VH CDR3, as well as VL CDR1, VL CDR2 and VL CDR3 are amino acid sequences listed below as options:
(a) VH CDR1 = DVAMQ, DTLMQ, DVAMG, DTYMG, DTYMQ or DVLMG;
(b) VH CDR2 = RIDPANGNTKYDPKFQA, RITYANGGTKADPKFNS, RIDPMGGNTKYDPKFQA, RIDPANGNTKYDPSQFA, RIDPMGGGTKADPKFNS, RISPANSTTKADPSQFA or RISPANSTTKADPKFQA;
(c) VH CDR3 = FGGYVRFDY, GCGYVRFYY, FGGYVRFYY, GCGYNRFDY, FGGYNRFDC, GCGYNRFDC or GCGKFRTDC;
(d) VL CDR1 = KSTSSLLSSNRNIHLLA, KSSQSLLKSRTRKHYLA, KSSQSLLSSRTRKHYLA, KSSASLLKSNRNIHLLA, KSSASLLSSRTRKHYLA or KSTSSLLSSNRKHYLLA;
(e) VL CDR2 = WASTRES, WATSRAS, WQSSRAS, WATSLDS, WQSSLEA or WASKRES; and
(f) VL CDR3 = KQGYNLWT, KQGYNLNT, KQYNALWT, KQYLNGNA, KQSYNLWT, KQYLNLWT, KQSYNGNA or KQSYNTWI.

3. The anti-soluble CD14 subtype monoclonal antibody or the antigen-binding antibody fragment thereof of claim 1 or 2, wherein
the anti-soluble CD14 subtype monoclonal antibody or the antigen-binding antibody fragment thereof comprises VH CDR1, VH CDR2 and VH CDR3, as well as VL CDR1, VL CDR2 and VL CDR3, which is any one of following 1) to 50):
1) VH CDR1 composed of an amino acid sequence of SEQ ID NO: 44, VH CDR2 composed of an amino acid sequence of SEQ ID NO: 50, VH CDR3 composed of an amino acid sequence of SEQ ID NO: 61, VL CDR1 composed of an amino acid sequence of SEQ ID NO: 69, VL CDR2 composed of an amino acid sequence of SEQ ID NO: 75, and VL CDR3 composed of an amino acid sequence of SEQ ID NO: 76;
2) VH CDR1 composed of an amino acid sequence of SEQ ID NO: 44, VH CDR2 composed of an amino acid sequence of SEQ ID NO: 51, VH CDR3 composed of an amino acid sequence of SEQ ID NO: 57, VL CDR1 composed of an amino acid sequence of SEQ ID NO: 64, VL CDR2 composed of an amino acid sequence of SEQ ID NO: 73, and VL CDR3 composed of an amino acid sequence of SEQ ID NO: 78;
3) VH CDR1 composed of an amino acid sequence of SEQ ID NO: 44, VH CDR2 composed of an amino acid sequence of SEQ ID NO: 53, VH CDR3 composed of an amino acid sequence of SEQ ID NO: 59, VL CDR1 composed of an amino acid sequence of SEQ ID NO: 67, VL CDR2 composed of an amino acid sequence of SEQ ID NO: 71, and VL CDR3 composed of an amino acid sequence of SEQ ID NO: 80;
4) VH CDR1 composed of an amino acid sequence of SEQ ID NO: 44, VH CDR2 composed of an amino acid sequence of SEQ ID NO: 53, VH CDR3 composed of an amino acid sequence of SEQ ID NO: 57, VL CDR1 composed of an amino acid sequence of SEQ ID NO: 68, VL CDR2 composed of an amino acid sequence of SEQ ID NO: 73, and VL CDR3 composed of an amino acid sequence of SEQ ID NO: 79;
5) VH CDR1 composed of an amino acid sequence of SEQ ID NO: 44, VH CDR2 composed of an amino acid sequence of SEQ ID NO: 54, VH CDR3 composed of an amino acid sequence of SEQ ID NO: 61, VL CDR1 composed of an amino acid sequence of SEQ ID NO: 66, VL CDR2 composed of an amino acid sequence of SEQ ID NO: 75, and VL CDR3 composed of an amino acid sequence of SEQ ID NO: 82;
6) VH CDR1 composed of an amino acid sequence of SEQ ID NO: 44, VH CDR2 composed of an amino acid sequence of SEQ ID NO: 54, VH CDR3 composed of an amino acid sequence of SEQ ID NO: 57, VL CDR1 composed of an amino acid sequence of SEQ ID NO: 64, VL CDR2 composed of an amino acid sequence of SEQ ID NO: 75, and VL CDR3 composed of an amino acid sequence of SEQ ID NO: 76;
7) VH CDR1 composed of an amino acid sequence of SEQ ID NO: 44, VH CDR2 composed of an amino acid sequence of SEQ ID NO: 56, VH CDR3 composed of an amino acid sequence of SEQ ID NO: 58, VL CDR1 composed of an amino acid sequence of SEQ ID NO: 68, VL CDR2 composed of an amino acid sequence of SEQ ID NO: 74, and VL CDR3 composed of an amino acid sequence of SEQ ID NO: 80;
8) VH CDR1 composed of an amino acid sequence of SEQ ID NO: 44, VH CDR2 composed of an amino acid sequence of SEQ ID NO: 56, VH CDR3 composed of an amino acid sequence of SEQ ID NO: 59, VL CDR1 composed of an amino acid sequence of SEQ ID NO: 66, VL CDR2 composed of an amino acid sequence of SEQ ID NO: 71, and VL CDR3 composed of an amino acid sequence of SEQ ID NO: 80;
9) VH CDR1 composed of an amino acid sequence of SEQ ID NO: 45, VH CDR2 composed of an amino acid sequence of SEQ ID NO: 50, VH CDR3 composed of an amino acid sequence of SEQ ID NO: 59, VL CDR1 composed of an amino acid sequence of SEQ ID NO: 64, VL CDR2 composed of an amino acid sequence of SEQ ID NO: 75, and VL CDR3 composed of an amino acid sequence of SEQ ID NO: 76;
10) VH CDR1 composed of an amino acid sequence of SEQ ID NO: 45, VH CDR2 composed of an amino acid sequence of SEQ ID NO: 51, VH CDR3 composed of an amino acid sequence of SEQ ID NO: 58, VL CDR1 composed of an amino acid sequence of SEQ ID NO: 66, VL CDR2 composed of an amino acid sequence of SEQ ID NO: 71, and VL CDR3 composed of an amino acid sequence of SEQ ID NO: 80;
11) VH CDR1 composed of an amino acid sequence of SEQ ID NO: 45, VH CDR2 composed of an amino acid sequence of SEQ ID NO: 51, VH CDR3 composed of an amino acid sequence of SEQ ID NO: 60, VL CDR1 composed of an amino acid sequence of SEQ ID NO: 67, VL CDR2 composed of an amino acid sequence of SEQ ID NO: 70, and VL CDR3 composed of an amino acid sequence of SEQ ID NO: 79;
12) VH CDR1 composed of an amino acid sequence of SEQ ID NO: 45, VH CDR2 composed of an amino acid sequence of SEQ ID NO: 52, VH CDR3 composed of an amino acid sequence of SEQ ID NO: 60, VL CDR1 composed of an amino acid sequence of SEQ ID NO: 66, VL CDR2 composed of an amino acid sequence of SEQ ID NO: 70, and VL CDR3 composed of an amino acid sequence of SEQ ID NO: 76;
13) VH CDR1 composed of an amino acid sequence of SEQ ID NO: 45, VH CDR2 composed of an amino acid sequence of SEQ ID NO: 53, VH CDR3 composed of an amino acid sequence of SEQ ID NO: 58, VL CDR1 composed of an amino acid sequence of SEQ ID NO: 69, VL CDR2 composed of an amino acid sequence of SEQ ID NO: 73, and VL CDR3 composed of an amino acid sequence of SEQ ID NO: 82;
14) VH CDR1 composed of an amino acid sequence of SEQ ID NO: 45, VH CDR2 composed of an amino acid sequence of SEQ ID NO: 53, VH CDR3 composed of an amino acid sequence of SEQ ID NO: 57, VL CDR1 composed of an amino acid sequence of SEQ ID NO: 65, VL CDR2 composed of an amino acid sequence of SEQ ID NO: 71, and VL CDR3 composed of an amino acid sequence of SEQ ID NO: 77;
15) VH CDR1 composed of an amino acid sequence of SEQ ID NO: 45, VH CDR2 composed of an amino acid sequence of SEQ ID NO: 54, VH CDR3 composed of an amino acid sequence of SEQ ID NO: 59, VL CDR1 composed of an amino acid sequence of SEQ ID NO: 66, VL CDR2 composed of an amino acid sequence of SEQ ID NO: 75, and VL CDR3 composed of an amino acid sequence of SEQ ID NO: 81;
16) VH CDR1 composed of an amino acid sequence of SEQ ID NO: 45, VH CDR2 composed of an amino acid sequence of SEQ ID NO: 55, VH CDR3 composed of an amino acid sequence of SEQ ID NO: 57, VL CDR1 composed of an amino acid sequence of SEQ ID NO: 68, VL CDR2 composed of an amino acid sequence of SEQ ID NO: 73, and VL CDR3 composed of an amino acid sequence of SEQ ID NO: 78;
17) VH CDR1 composed of an amino acid sequence of SEQ ID NO: 45, VH CDR2 composed of an amino acid sequence of SEQ ID NO: 56, VH CDR3 composed of an amino acid sequence of SEQ ID NO: 62, VL CDR1 composed of an amino acid sequence of SEQ ID NO: 69, VL CDR2 composed of an amino acid sequence of SEQ ID NO: 71, and VL CDR3 composed of an amino acid sequence of SEQ ID NO: 78;
18) VH CDR1 composed of an amino acid sequence of SEQ ID NO: 45, VH CDR2 composed of an amino acid sequence of SEQ ID NO: 56, VH CDR3 composed of an amino acid sequence of SEQ ID NO: 59, VL CDR1 composed of an amino acid sequence of SEQ ID NO: 69, VL CDR2 composed of an amino acid sequence of SEQ ID NO: 71, and VL CDR3 composed of an amino acid sequence of SEQ ID NO: 83;
19) VH CDR1 composed of an amino acid sequence of SEQ ID NO: 46, VH CDR2 composed of an amino acid sequence of SEQ ID NO: 50, VH CDR3 composed of an amino acid sequence of SEQ ID NO: 62, VL CDR1 composed of an amino acid sequence of SEQ ID NO: 69, VL CDR2 composed of an amino acid sequence of SEQ ID NO: 70, and VL CDR3 composed of an amino acid sequence of SEQ ID NO: 81;
20) VH CDR1 composed of an amino acid sequence of SEQ ID NO: 46, VH CDR2 composed of an amino acid sequence of SEQ ID NO: 51, VH CDR3 composed of an amino acid sequence of SEQ ID NO: 58, VL CDR1 composed of an amino acid sequence of SEQ ID NO: 67, VL CDR2 composed of an amino acid sequence of SEQ ID NO: 73, and VL CDR3 composed of an amino acid sequence of SEQ ID NO: 83;
21) VH CDR1 composed of an amino acid sequence of SEQ ID NO: 46, VH CDR2 composed of an amino acid sequence of SEQ ID NO: 52, VH CDR3 composed of an amino acid sequence of SEQ ID NO: 61, VL CDR1 composed of an amino acid sequence of SEQ ID NO: 68, VL CDR2 composed of an amino acid sequence of SEQ ID NO: 71, and VL CDR3 composed of an amino acid sequence of SEQ ID NO: 83;
22) VH CDR1 composed of an amino acid sequence of SEQ ID NO: 46, VH CDR2 composed of an amino acid sequence of SEQ ID NO: 52, VH CDR3 composed of an amino acid sequence of SEQ ID NO: 60, VL CDR1 composed of an amino acid sequence of SEQ ID NO: 67, VL CDR2 composed of an amino acid sequence of SEQ ID NO: 74, and VL CDR3 composed of an amino acid sequence of SEQ ID NO: 81;
23) VH CDR1 composed of an amino acid sequence of SEQ ID NO: 46, VH CDR2 composed of an amino acid sequence of SEQ ID NO: 52, VH CDR3 composed of an amino acid sequence of SEQ ID NO: 59, VL CDR1 composed of an amino acid sequence of SEQ ID NO: 69, VL CDR2 composed of an amino acid sequence of SEQ ID NO: 75, and VL CDR3 composed of an amino acid sequence of SEQ ID NO: 83;
24) VH CDR1 composed of an amino acid sequence of SEQ ID NO: 46, VH CDR2 composed of an amino acid sequence of SEQ ID NO: 53, VH CDR3 composed of an amino acid sequence of SEQ ID NO: 60, VL CDR1 composed of an amino acid sequence of SEQ ID NO: 64, VL CDR2 composed of an amino acid sequence of SEQ ID NO: 72, and VL CDR3 composed of an amino acid sequence of SEQ ID NO: 76;
25) VH CDR1 composed of an amino acid sequence of SEQ ID NO: 46, VH CDR2 composed of an amino acid sequence of SEQ ID NO: 54, VH CDR3 composed of an amino acid sequence of SEQ ID NO: 63, VL CDR1 composed of an amino acid sequence of SEQ ID NO: 66, VL CDR2 composed of an amino acid sequence of SEQ ID NO: 71, and VL CDR3 composed of an amino acid sequence of SEQ ID NO: 79;
26) VH CDR1 composed of an amino acid sequence of SEQ ID NO: 46, VH CDR2 composed of an amino acid sequence of SEQ ID NO: 55, VH CDR3 composed of an amino acid sequence of SEQ ID NO: 57, VL CDR1 composed of an amino acid sequence of SEQ ID NO: 64, VL CDR2 composed of an amino acid sequence of SEQ ID NO: 75, and VL CDR3 composed of an amino acid sequence of SEQ ID NO: 77;
27) VH CDR1 composed of an amino acid sequence of SEQ ID NO: 46, VH CDR2 composed of an amino acid sequence of SEQ ID NO: 56, VH CDR3 composed of an amino acid sequence of SEQ ID NO: 63, VL CDR1 composed of an amino acid sequence of SEQ ID NO: 65, VL CDR2 composed of an amino acid sequence of SEQ ID NO: 73, and VL CDR3 composed of an amino acid sequence of SEQ ID NO: 79;
28) VH CDR1 composed of an amino acid sequence of SEQ ID NO: 46, VH CDR2 composed of an amino acid sequence of SEQ ID NO: 56, VH CDR3 composed of an amino acid sequence of SEQ ID NO: 58, VL CDR1 composed of an amino acid sequence of SEQ ID NO: 65, VL CDR2 composed of an amino acid sequence of SEQ ID NO: 75, and VL CDR3 composed of an amino acid sequence of SEQ ID NO: 82;
29) VH CDR1 composed of an amino acid sequence of SEQ ID NO: 47, VH CDR2 composed of an amino acid sequence of SEQ ID NO: 51, VH CDR3 composed of an amino acid sequence of SEQ ID NO: 57, VL CDR1 composed of an amino acid sequence of SEQ ID NO: 65, VL CDR2 composed of an amino acid sequence of SEQ ID NO: 72, and VL CDR3 composed of an amino acid sequence of SEQ ID NO: 77;
30) VH CDR1 composed of an amino acid sequence of SEQ ID NO: 47, VH CDR2 composed of an amino acid sequence of SEQ ID NO: 52, VH CDR3 composed of an amino acid sequence of SEQ ID NO: 62, VL CDR1 composed of an amino acid sequence of SEQ ID NO: 65, VL CDR2 composed of an amino acid sequence of SEQ ID NO: 74, and VL CDR3 composed of an amino acid sequence of SEQ ID NO: 77;
31) VH CDR1 composed of an amino acid sequence of SEQ ID NO: 47, VH CDR2 composed of an amino acid sequence of SEQ ID NO: 53, VH CDR3 composed of an amino acid sequence of SEQ ID NO: 61, VL CDR1 composed of an amino acid sequence of SEQ ID NO: 68, VL CDR2 composed of an amino acid sequence of SEQ ID NO: 72, and VL CDR3 composed of an amino acid sequence of SEQ ID NO: 77;
32) VH CDR1 composed of an amino acid sequence of SEQ ID NO: 47, VH CDR2 composed of an amino acid sequence of SEQ ID NO: 53, VH CDR3 composed of an amino acid sequence of SEQ ID NO: 57, VL CDR1 composed of an amino acid sequence of SEQ ID NO: 67, VL CDR2 composed of an amino acid sequence of SEQ ID NO: 71, and VL CDR3 composed of an amino acid sequence of SEQ ID NO: 77;
33) VH CDR1 composed of an amino acid sequence of SEQ ID NO: 47, VH CDR2 composed of an amino acid sequence of SEQ ID NO: 55, VH CDR3 composed of an amino acid sequence of SEQ ID NO: 60, VL CDR1 composed of an amino acid sequence of SEQ ID NO: 69, VL CDR2 composed of an amino acid sequence of SEQ ID NO: 75, and VL CDR3 composed of an amino acid sequence of SEQ ID NO: 83;
34) VH CDR1 composed of an amino acid sequence of SEQ ID NO: 47, VH CDR2 composed of an amino acid sequence of SEQ ID NO: 55, VH CDR3 composed of an amino acid sequence of SEQ ID NO: 58, VL CDR1 composed of an amino acid sequence of SEQ ID NO: 64, VL CDR2 composed of an amino acid sequence of SEQ ID NO: 73, and VL CDR3 composed of an amino acid sequence of SEQ ID NO: 83;
35) VH CDR1 composed of an amino acid sequence of SEQ ID NO: 47, VH CDR2 composed of an amino acid sequence of SEQ ID NO: 56, VH CDR3 composed of an amino acid sequence of SEQ ID NO: 63, VL CDR1 composed of an amino acid sequence of SEQ ID NO: 65, VL CDR2 composed of an amino acid sequence of SEQ ID NO: 70, and VL CDR3 composed of an amino acid sequence of SEQ ID NO: 82;
36) VH CDR1 composed of an amino acid sequence of SEQ ID NO: 48, VH CDR2 composed of an amino acid sequence of SEQ ID NO: 50, VH CDR3 composed of an amino acid sequence of SEQ ID NO: 61, VL CDR1 composed of an amino acid sequence of SEQ ID NO: 66, VL CDR2 composed of an amino acid sequence of SEQ ID NO: 70, and VL CDR3 composed of an amino acid sequence of SEQ ID NO: 80;
37) VH CDR1 composed of an amino acid sequence of SEQ ID NO: 48, VH CDR2 composed of an amino acid sequence of SEQ ID NO: 50, VH CDR3 composed of an amino acid sequence of SEQ ID NO: 58, VL CDR1 composed of an amino acid sequence of SEQ ID NO: 66, VL CDR2 composed of an amino acid sequence of SEQ ID NO: 70, and VL CDR3 composed of an amino acid sequence of SEQ ID NO: 82;
38) VH CDR1 composed of an amino acid sequence of SEQ ID NO: 48, VH CDR2 composed of an amino acid sequence of SEQ ID NO: 51, VH CDR3 composed of an amino acid sequence of SEQ ID NO: 62, VL CDR1 composed of an amino acid sequence of SEQ ID NO: 64, VL CDR2 composed of an amino acid sequence of SEQ ID NO: 75, and VL CDR3 composed of an amino acid sequence of SEQ ID NO: 81;
39) VH CDR1 composed of an amino acid sequence of SEQ ID NO: 48, VH CDR2 composed of an amino acid sequence of SEQ ID NO: 52, VH CDR3 composed of an amino acid sequence of SEQ ID NO: 61, VL CDR1 composed of an amino acid sequence of SEQ ID NO: 67, VL CDR2 composed of an amino acid sequence of SEQ ID NO: 72, and VL CDR3 composed of an amino acid sequence of SEQ ID NO: 79;
40) VH CDR1 composed of an amino acid sequence of SEQ ID NO: 48, VH CDR2 composed of an amino acid sequence of SEQ ID NO: 52, VH CDR3 composed of an amino acid sequence of SEQ ID NO: 59, VL CDR1 composed of an amino acid sequence of SEQ ID NO: 67, VL CDR2 composed of an amino acid sequence of SEQ ID NO: 72, and VL CDR3 composed of an amino acid sequence of SEQ ID NO: 80;
41) VH CDR1 composed of an amino acid sequence of SEQ ID NO: 48, VH CDR2 composed of an amino acid sequence of SEQ ID NO: 53, VH CDR3 composed of an amino acid sequence of SEQ ID NO: 61, VL CDR1 composed of an amino acid sequence of SEQ ID NO: 65, VL CDR2 composed of an amino acid sequence of SEQ ID NO: 71, and VL CDR3 composed of an amino acid sequence of SEQ ID NO: 80;
42) VH CDR1 composed of an amino acid sequence of SEQ ID NO: 48, VH CDR2 composed of an amino acid sequence of SEQ ID NO: 54, VH CDR3 composed of an amino acid sequence of SEQ ID NO: 58, VL CDR1 composed of an amino acid sequence of SEQ ID NO: 66, VL CDR2 composed of an amino acid sequence of SEQ ID NO: 71, and VL CDR3 composed of an amino acid sequence of SEQ ID NO: 82;
43) VH CDR1 composed of an amino acid sequence of SEQ ID NO: 48, VH CDR2 composed of an amino acid sequence of SEQ ID NO: 54, VH CDR3 composed of an amino acid sequence of SEQ ID NO: 57, VL CDR1 composed of an amino acid sequence of SEQ ID NO: 64, VL CDR2 composed of an amino acid sequence of SEQ ID NO: 72, and VL CDR3 composed of an amino acid sequence of SEQ ID NO: 77;
44) VH CDR1 composed of an amino acid sequence of SEQ ID NO: 48, VH CDR2 composed of an amino acid sequence of SEQ ID NO: 55, VH CDR3 composed of an amino acid sequence of SEQ ID NO: 58, VL CDR1 composed of an amino acid sequence of SEQ ID NO: 69, VL CDR2 composed of an amino acid sequence of SEQ ID NO: 75, and VL CDR3 composed of an amino acid sequence of SEQ ID NO: 81;
45) VH CDR1 composed of an amino acid sequence of SEQ ID NO: 48, VH CDR2 composed of an amino acid sequence of SEQ ID NO: 55, VH CDR3 composed of an amino acid sequence of SEQ ID NO: 63, VL CDR1 composed of an amino acid sequence of SEQ ID NO: 64, VL CDR2 composed of an amino acid sequence of SEQ ID NO: 70, and VL CDR3 composed of an amino acid sequence of SEQ ID NO: 76;
46) VH CDR1 composed of an amino acid sequence of SEQ ID NO: 49, VH CDR2 composed of an amino acid sequence of SEQ ID NO: 50, VH CDR3 composed of an amino acid sequence of SEQ ID NO: 60, VL CDR1 composed of an amino acid sequence of SEQ ID NO: 66, VL CDR2 composed of an amino acid sequence of SEQ ID NO: 72, and VL CDR3 composed of an amino acid sequence of SEQ ID NO: 81;
47) VH CDR1 composed of an amino acid sequence of SEQ ID NO: 49, VH CDR2 composed of an amino acid sequence of SEQ ID NO: 51, VH CDR3 composed of an amino acid sequence of SEQ ID NO: 60, VL CDR1 composed of an amino acid sequence of SEQ ID NO: 66, VL CDR2 composed of an amino acid sequence of SEQ ID NO: 74, and VL CDR3 composed of an amino acid sequence of SEQ ID NO: 83;
48) VH CDR1 composed of an amino acid sequence of SEQ ID NO: 49, VH CDR2 composed of an amino acid sequence of SEQ ID NO: 51, VH CDR3 composed of an amino acid sequence of SEQ ID NO: 62, VL CDR1 composed of an amino acid sequence of SEQ ID NO: 65, VL CDR2 composed of an amino acid sequence of SEQ ID NO: 70, and VL CDR3 composed of an amino acid sequence of SEQ ID NO: 81;
49) VH CDR1 composed of an amino acid sequence of SEQ ID NO: 49, VH CDR2 composed of an amino acid sequence of SEQ ID NO: 52, VH CDR3 composed of an amino acid sequence of SEQ ID NO: 59, VL CDR1 composed of an amino acid sequence of SEQ ID NO: 66, VL CDR2 composed of an amino acid sequence of SEQ ID NO: 70, and VL CDR3 composed of an amino acid sequence of SEQ ID NO: 79; or
50) VH CDR1 composed of an amino acid sequence of SEQ ID NO: 49, VH CDR2 composed of an amino acid sequence of SEQ ID NO: 53, VH CDR3 composed of an amino acid sequence of SEQ ID NO: 60, VL CDR1 composed of an amino acid sequence of SEQ ID NO: 68, VL CDR2 composed of an amino acid sequence of SEQ ID NO: 71, and VL CDR3 composed of an amino acid sequence of SEQ ID NO: 77.

4. The anti-soluble CD14 subtype monoclonal antibody or the antigen-binding antibody fragment thereof of claim 1, comprising:
(a) VH comprising VH CDR1 composed of an amino acid sequence of SEQ ID NO: 44, VH CDR2 composed of an amino acid sequence of SEQ ID NO: 54, and VH CDR3 composed of an amino acid sequence of SEQ ID NO: 61, and VL comprising VL CDR1 composed of an amino acid sequence of SEQ ID NO: 66, VL CDR2 composed of an amino acid sequence of SEQ ID NO: 75, and VL CDR3 composed of an amino acid sequence of SEQ ID NO: 82;
(b) VH comprising VH CDR1 composed of an amino acid sequence of SEQ ID NO: 45, VH CDR2 composed of an amino acid sequence of SEQ ID NO: 53, and VH CDR3 composed of an amino acid sequence of SEQ ID NO: 57, and VL comprising VL CDR1 composed of an amino acid sequence of SEQ ID NO: 65, VL CDR2 composed of an amino acid sequence of SEQ ID NO: 71, and VL CDR3 composed of an amino acid sequence of SEQ ID NO: 77;
(c) VH comprising VH CDR1 composed of an amino acid sequence of SEQ ID NO: 46, VH CDR2 composed of an amino acid sequence of SEQ ID NO: 52, and VH CDR3 composed of an amino acid sequence of SEQ ID NO: 61, and VL comprising VL CDR1 composed of an amino acid sequence of SEQ ID NO: 68, VL CDR2 composed of an amino acid sequence of SEQ ID NO: 71, and VL CDR3 composed of an amino acid sequence of SEQ ID NO: 83;
(d) VH comprising VH CDR1 composed of an amino acid sequence of SEQ ID NO: 48, VH CDR2 composed of an amino acid sequence of SEQ ID NO: 50, and VH CDR3 composed of an amino acid sequence of SEQ ID NO: 61, and VL comprising VL CDR1 composed of an amino acid sequence of SEQ ID NO: 66, VL CDR2 composed of an amino acid sequence of SEQ ID NO: 70, and VL CDR3 composed of an amino acid sequence of SEQ ID NO: 80;
(e) VH comprising VH CDR1 composed of an amino acid sequence of SEQ ID NO: 48, VH CDR2 composed of an amino acid sequence of SEQ ID NO: 52, and VH CDR3 composed of an amino acid sequence of SEQ ID NO: 61, and VL comprising VL CDR1 composed of an amino acid sequence of SEQ ID NO: 67, VL CDR2 composed of an amino acid sequence of SEQ ID NO: 72, and VL CDR3 composed of an amino acid sequence of SEQ ID NO: 79; or
(f) VH comprising VH CDR1 composed of an amino acid sequence of SEQ ID NO: 49, VH CDR2 composed of an amino acid sequence of SEQ ID NO: 51, and VH CDR3 composed of an amino acid sequence of SEQ ID NO: 62, and VL comprising VL CDR1 composed of an amino acid sequence of SEQ ID NO: 65, VL CDR2 composed of an amino acid sequence of SEQ ID NO: 70, and VL CDR3 composed of an amino acid sequence of SEQ ID NO: 81.

5. The anti-soluble CD14 subtype monoclonal antibody or the antigen-binding antibody fragment thereof of claim 1, wherein the soluble CD14 monoclonal antibody or the antigen-binding antibody fragment thereof is an anti-soluble CD14 subtype monoclonal antibody or an antigen-binding antibody fragment thereof secreted and produced by a hybridoma cell strain with the deposit number of CGMCC NO. 19395.

6. The anti-soluble CD14 subtype monoclonal antibody or the antigen-binding antibody fragment thereof of any one of claims 1 to 5,
wherein the anti-soluble CD14 subtype monoclonal antibody or the antigen-binding antibody fragment thereof does not specifically bind to a soluble CD14 with a high molecular weight.

7. The anti-soluble CD14 subtype monoclonal antibody or the antigen-binding antibody fragment thereof of any one of claims 1 to 6,
wherein the antigen-binding antibody fragment thereof is an antigen-binding antibody fragment selected from a group consisting of Fab, Fab', F(ab')2, a single-chain antibody (scFV), a dimerized V region (a dimeric antibody), a disulfide stabilized V region (dsFv), sc(Fv)2, a CDR-containing polypeptide, a heavy chain variable region-containing polypeptide, and a light chain variable region-containing polypeptide.

8. A kit for detecting a soluble CD14 subtype, wherein the kit comprises at least the anti-soluble CD14 subtype monoclonal antibody or the antigen-binding antibody fragment thereof of any one of claims 1 to 7 as a first monoclonal antibody or an antigen-binding antibody fragment thereof.

9. The kit of claim 8, wherein the kit further comprises a second monoclonal antibody or an antigen-binding antibody fragment thereof,
the second monoclonal antibody or the antigen-binding antibody fragment thereof specifically recognizing an epitope composed of an amino acid sequence of SEQ ID No. 42, wherein
the second monoclonal antibody or the antigen-binding antibody fragment thereof comprises:
(i) heavy chain variable region (VH) complementarity determining regions (CDRs):
VH CDR1: X₁X₂X₃MX₄;
VH CDR2: YIX₅X₆ADX₇; and
VH CDR3: X₈X₉X₁₀AX₁₁;
and
(ii) light chain variable region (VL) complementarity determining regions (CDRs):
VL CDR1: KX₁₂X₁₃X₁₄N;
VL CDR2: LX₁₅X₁₆; and
VL CDR3: VX₁₇X₁₈X₁₉;
where X₁ to X₁₉ are one or more of amino acid sequences listed below as options:
X₁ = any one of amino acids;
X₂ = F or V; X₃ = A, E or K; X₄ = A or L;
X₅ = SYMGS, SSGSS, AYTMY, TYSGS or SSKSS;
X₆ = GAYY, TIYY, AKYY, TKAS or SNLA;
X₇ = AKLG, TVKG, SYTA, MTKG or YGNT;
X₈ = A or Q; X₉ = G or Q; X₁₀ = Q or F; X₁₁ = M, Y or V;
X₁₂ = YYAS, SSAT, SSQS or SGSS;
X₁₃ = AAKL, LLAT, LLYS or KAAS;
X₁₄ = YRNIKL, TWAKGN, NGKTYL or YTNGAL;
X₁₅ = VQS, KTS, QTS or VSK;
X₁₆ = LAS, LDS, LTA or DSK;
X₁₇ = G, A, S or Q;
X₁₈ = GTH, GNT, GTA or TAI;
X₁₉ = FITA, FPRT, YGHV or NYGH.

10. The kit of claim 9, wherein
the second monoclonal antibody or the antigen-binding antibody fragment thereof comprises VH CDR1, VH CDR2 and VH CDR3, as well as VL CDR1, VL CDR2 and VL CDR3,
wherein VH CDR1, VH CDR2 and VH CDR3, as well as VL CDR1, VL CDR2 and VL CDR3 are amino acid sequences listed below as options:
(a) VH CDR1 = SVAML, SVEML, SFAML, SFAMA, SVKML or SFKMA;
(b) VH CDR2 = YISYMGSTIYYADAKLG, YISSKSSSNLAADAKLG, YISSGSSTIYYADTVKG, YISSGSSTIYYADMTKG, YITYSGSGAYYADYGNT, YISYMGSTIYYADTVKG or YISSKSSTIYYADTVKG;
(c) VH CDR3 = AGFAY, QQFAY, QGFAY, QGFAM, QQVAY, AGFAM or AGFAY;
(d) VL CDR1 = KSSQSLLYSNGKTYLN, KYYASAAKLYRNIKLN, KYYASLLATYRNIKLN, KYYASLLATTWAKGNN, KSSQSLLYSTWAKGNN or KSSQSLLYSYRNIKLN;
(e) VL CDR2 = LVQSLDS, LVSKLTA, LVSKLDS, LVQSDSK, LQTSDSK or LVSKLAS; and
(f) VL CDR3 = VQGTHNYGH, VGGTHFPRT, VGTAIFPRT, VGGTHFITA, VSGTHFPRT, VQGTHFPRT, VQTAIFPRT, VQGTHYGHV or VQGNTFITA.

11. The kit of claim 9, wherein
the second monoclonal antibody or the antigen-binding antibody fragment thereof comprises VH CDR1, VH CDR2 and VH CDR3, as well as VL CDR1, VL CDR2 and VL CDR3,
wherein VH CDR1, VH CDR2 and VH CDR3, as well as VL CDR1, VL CDR2 and VL CDR3 are amino acid sequences listed below as options:
(a) VH CDR1 = SVAML, SVEML, SFAML, SFAMA, SVKML or SFKMA;
(b) VH CDR2 = YISYMGSTIYYADAKLG, YISSKSSSNLAADAKLG, YISSGSSTIYYADTVKG or YISSGSSTIYYADMTKG;
(c) VH CDR3 = AGFAY, QQFAY, QGFAY or QGFAM;
(d) VL CDR1 = KYYASAAKLYRNIKLN, KYYASLLATYRNIKLN, KSSQSLLYSNGKTYLN or KYYASLLATTWAKGNN;
(e) VL CDR2 = LVQSLDS, LVSKLDS, LVSKLTA or LVQSDSK; and
(f) VL CDR3 = VQGTHNYGH, VGGTHFPRT, VQGTHFPRT, VGTAIFPRT or VGGTHFITA.

12. The kit of any one of claims 9 to 11, wherein
the second monoclonal antibody or the antigen-binding antibody fragment thereof is an anti-soluble CD14 subtype monoclonal antibody or an antigen-binding antibody fragment thereof secreted and produced by a hybridoma cell strain with the deposit number of CGMCC NO. 18536.

13. The kit of any one of claims 9 to 12, wherein the second monoclonal antibody or the antigen-binding antibody fragment thereof is used as a capture antibody and the first monoclonal antibody or the antigen-binding antibody fragment thereof is used as a labelled detection antibody, and the capture antibody is coated on a solid phase carrier and used to capture the soluble CD14 subtype in a sample to be tested.

14. The kit of claim 13, wherein the capture antibody is coated on a surface of a magnetic bead, especially the surface of a superparamagnetic bead.

15. A method for detecting a soluble CD14 subtype, comprising:
mixing a sample to be tested with a solid phase support coated with a capture antibody, so that the capture antibody coated on the solid phase support binds to the soluble CD14 subtype in the sample to be tested;
washing the obtained mixture to remove unbound substances;
adding a labelled detection antibody to the washed mixture and mixing evenly, so that the detection antibody binds to the soluble CD14 subtype bound on the capture antibody to form a sandwich complex;
washing the sandwich complex to remove unbound substances;
adding a detection substrate to the washed sandwich complex to detect a concentration of the soluble CD14 subtype in the sample;
wherein the capture antibody or the detection antibody is the anti-soluble CD14 subtype monoclonal antibody or the antigen-binding antibody fragment thereof of any one of claims 1 to 7 as a first monoclonal antibody or an antigen-binding antibody fragment.

16. The method of claim 15, wherein the detection antibody is the anti-soluble CD14 subtype monoclonal antibody or the antigen-binding antibody fragment thereof of any one of claims 1 to 7 as a first monoclonal antibody or an antigen-binding antibody fragment thereof.

17. The method of claim 16, wherein
the capture antibody is an anti-soluble CD14 subtype monoclonal antibody or an antigen-binding antibody fragment thereof described below as a second monoclonal antibody or an antigen-binding antibody fragment thereof,
the second monoclonal antibody or the antigen-binding antibody fragment thereof specifically recognizing an epitope composed of amino acid sequence of SEQ ID No. 42, wherein
the second monoclonal antibody or the antigen-binding antibody fragment thereof comprises:
(i) heavy chain variable region (VH) complementarity determining regions (CDRs):
VH CDR1: X₁X₂X₃MX₄;
VH CDR2: YIX₅X₆ADX₇; and
VH CDR3: X₈X₉X₁₀AX₁₁;
and
(ii) light chain variable region (VL) complementarity determining regions (CDRs):
VL CDR1: KX₁₂X₁₃X₁₄N;
VL CDR2: LX₁₅X₁₆; and
VL CDR3: VX₁₇X₁₈X₁₉;
where X₁ to X₁₉ are one or more of amino acid sequences listed below as options:
X₁ = any one of amino acids;
X₂ = F or V; X₃ = A, E or K; X₄ = A or L;
X₅ = SYMGS, SSGSS, AYTMY, TYSGS or SSKSS;
X₆ = GAYY, TIYY, AKYY, TKAS or SNLA;
X₇ = AKLG, TVKG, SYTA, MTKG or YGNT;
X₈ = A or Q; X₉ = G or Q; X₁₀ = Q or F; X₁₁ = M, Y or V;
X₁₂ = YYAS, SSAT, SSQS or SGSS;
X₁₃ = AAKL, LLAT, LLYS or KAAS;
X₁₄ = YRNIKL, TWAKGN, NGKTYL or YTNGAL;
X₁₅ = VQS, KTS, QTS or VSK;
X₁₆ = LAS, LDS, LTA or DSK;
X₁₇ = G, A, S or Q;
X₁₈ = GTH, GNT, GTA or TAI;
X₁₉ = FITA, FPRT, YGHV or NYGH.

18. The method of claim 17, wherein the second monoclonal antibody or the antigen-binding antibody fragment thereof is an anti-soluble CD 14 subtype monoclonal antibody or an antigen-binding antibody fragment thereof secreted and produced by a hybridoma cell strain with the deposit number of CGMCC NO. 18536.

19. The method of any one of claims 15 to 18, wherein the sample to be detected is a blood sample, especially a plasma sample.

20. Use of the anti-soluble CD14 subtype monoclonal antibody or the antigen-binding antibody fragment thereof of any one of claims 1 to 7 in the preparation of an analysis reagent for identifying whether a patient suffers from sepsis, wherein the anti-soluble CD14 subtype monoclonal antibody or the antigen-binding antibody fragment thereof of any one of claims 1 to 7 is used to detect a concentration of a soluble CD14 subtype in a blood sample from the patient, and an increase in the concentration of the soluble CD14 subtype relative to a reference level is associated with an increase in possibility that the patient suffers from sepsis.

21. The use of claim 20,
wherein the concentration of the soluble CD14 subtype in the blood sample from the patient is tested within 72 hours after the patient is suspected of suffering from sepsis.

22. An anti-soluble CD14 subtype monoclonal antibody or an antigen-binding antibody fragment thereof, which specifically recognizes an epitope composed of the amino acid sequence of SEQ ID No. 84, wherein the soluble CD14 subtype capture antibody is an anti-soluble CD14 subtype monoclonal antibody or the antigen-binding antibody fragment thereof secreted and produced by a hybridoma cell strain with the deposit number of CGMCC NO. 19395.
